# EUROPEAN PATENT APPLICATION

(11) **EP 4 393 505 A1**
(43) Date of publication of application: **03.07.2024**
(21) Application number: 22861612.4
(22) Date of filing: 12.08.2022
(51) Int. Cl.: A61K 39/187, A61P 31/20, C07K 14/005, C12N 15/82, A23K 20/147, A01H 1/06, A61K 39/00

(54) **VACCINE FOR PREVENTING AFRICAN SWINE FEVER, COMPRISING AFRICAN SWINE FEVER VIRUS-DERIVED ANTIGEN PROTEIN**

(30) Priority: 27.08.2021 KR 20210114014; 04.08.2022 KR 20220097525
(71) Applicant: Bioapplications Inc., Pohang-si, Gyeongsangbuk-do 37668 (KR)
(72) Inventor: KANG, Hyangju, Pohang-si, Gyeongsangbuk-do 37780 (KR); CHOI, Bo-Hwa, Pohang-si, Gyeongsangbuk-do 37655 (KR); SOHN, Eun-Ju, Pohang-si, Gyeongsangbuk-do 37668 (KR)
(74) Representative: Witthoff Jaekel Steinecke Patentanwälte PartG mbB
(86) International application number: PCT/KR2022/012142
(87) International publication number: WO 2023/027402

(57) **Abstract**

The present invention relates to: a recombinant vector comprising a nucleotide sequence of antigen protein(s) Lectin, CD2v, p72, p54, p30, p15, p35, E199L, and/or F317L of an African swine fever virus; a transformant transformed by means of the recombinant vector; and a vaccine composition for preventing African swine fever, comprising, as an active ingredient, African swine fever virus antigen protein(s) Lectin, CD2v, p72, p54, p30, p15, p35, E199L, and/or F317L, isolated from the transformant; and the like.

## Description

### [Technical Field]

The present invention relates to a vaccine for preventing African swine fever, including antigen proteins derived from African swine fever virus as active ingredients and the like.

The present invention claims priority based on Korean Patent Application No. 10-2021-0114014, filed on August 27, 2021, and Korean Patent Application No. 10-2022-0097525, filed on August 4, 2022, and the all contents disclosed in the specification and drawings of the corresponding applications are incorporated into this application.

### [Background Art]

African swine fever (ASF) is a swine infectious disease caused by infections with the African swine fever virus (ASFV) belonging to the family *Asfarviridae,* which was first reported in Kenya in 1921, and afterwards, there were reports of an outbreak in the sub-Saharan region, and since 2007, the outbreak has begun to expand to regions other than Africa, such as Georgia, Armenia and Azerbaijan, which are on the Black Sea coast. In particular, China accounts for about half of the world's pig breeding, and by ASF which first occurred in 2018, officially about 7 million pigs, and actually estimated about 200 million pigs, were killed and suffered enormous property damage. Although the ASF outbreak has since been brought under control, another ASF case was reported earlier this year in Northern China. Vietnam also had a recurrence of ASF this year and slaughtered about 36,000 pigs in 2021 alone and more than 6 million pigs from 2019 until May of this year, but new outbreak cases continue to be reported across the country. In addition, Malaysia, which had no cases of outbreak, also detected the ASF virus for the first time at the end of February this year.

In South Korea, the first ASF outbreak was reported in a pig farm in Paju in 2019, and the cases of infection continued to be reported in Gyeonggi-do and Gangwon-do. As of September 2020, African swine fever was confirmed in wild boars in Paju-si, Yeoncheon-gun and Pocheon-si in Gyeonggi-do, and it was confirmed at 9 locations in Gangwon-do, including Cheorwon-gun, Hwacheon-gun, Chuncheon-si, Yanggu-gun, Inje-gun and Goseong-gun. Among these, Hwacheon-si, Gangwon-do had the most cases with 285, followed by Yeoncheon-gun, Gyeonggi-do with 282 cases, and Paju-si, Gyeonggi-do with 98 cases. As such, in order to prevent the spread of ASF, wide-area fences were installed, and active wild boar capture and quarantine were implemented, and as a result, after an infection case at a pig farm in Hwacheon, Gangwon-do in October 2020, ASFV infection was not reported for a while, but recently (August 2021), another case of ASF was reported at a pig farm in Gangwon-do.

African swine fever is a high-risk infectious disease that has a mortality rate of 100% when infected in pigs, and since a vaccine for this disease has not been developed, pigs must be disposed of immediately upon outbreak, and it remains a very important task to prevent the same.

Meanwhile, the remarkable development of molecular biology and genetic engineering technology is applied to the field of plants, and efforts to produce useful physiologically active substances from plants are steadily continuing. The production of useful substances from plants can significantly reduce the production costs, and it is possible to exclude various contaminants (viruses, oncogenes, enterotoxins, etc.) that may arise from conventional popular methods (methods of synthesizing proteins from animal cells or microorganisms and isolating and purifying the same), and also, even at the commercialization stage, it has the advantage of being able to manage seed stock, unlike animal cells or microorganisms.

Accordingly, the inventors of the present invention have made diligent efforts to develop an antigen for preventing African swine fever, and as a result, the inventors of the present invention have developed a system that can express African swine fever virus antigen proteins in plants with high efficiency, and completed the present invention by confirming that among the above antigen proteins, the combination of specific proteins can prevent African swine fever more effectively than other ASFV antigen protein combinations.

### [Disclosure]

### [Technical Problem]

African swine fever virus is a giant double-stranded DNA virus that replicates in the cytoplasm of infected cells. The virus causes hemorrhagic fever with high mortality in pigs, and persistently infects its natural hosts, pigs, warthogs, river boars and the genus *Ornithodoros,* which may serve as vectors without signs of disease. Since this virus causes fatal hemorrhagic fever in pigs, it is an infectious disease that is very important to prevent before infection.

The present invention has been devised to solve the problems of prior art and the need to prevent African swine fever as described above, and the present invention is directed to providing a recombinant antigen protein derived from a recombinant African swine fever virus that not only enables efficient production by using a plant but also exhibits high immunogenicity, a vaccine composition including the same and the like.

However, the technical problems to be achieved by the present invention are not limited to the above-mentioned problems, and other problems that are not mentioned will be clearly understood by those skilled in the art from the description below.

### [Technical Solution]

The present invention provides a vaccine composition for preventing African swine fever, including a combination of African swine fever virus (ASFV) antigen proteins as an active ingredient, wherein the combination of ASFV antigen proteins is a combination of at least one selected form the group consisting of Lectin, CD2v, p72, p54 and p30 proteins, and wherein when the combination of ASFC antigen proteins includes the Lectin protein and the CD2v protein, the combination of ASFV antigen proteins consists of at least three antigen proteins.

In still another embodiment of the present invention, the vaccine composition may satisfy at least one feature selected from the group consisting of the following, but the present invention is not limited thereto:
(a) the Lectin protein includes the amino acid sequence of SEQ ID NO: 1;
(b) the CD2v protein includes the amino acid sequence of SEQ ID NO: 3;
(c) the p72 protein includes the amino acid sequence of SEQ ID NO: 5;
(d) the p54 protein includes the amino acid sequence of SEQ ID NO: 7; and
(e) the p30 protein includes the amino acid sequence of SEQ ID NO: 9.

In still another embodiment of the present invention, the ASFV antigen protein may be produced by using a recombinant vector, and the recombinant vector may include at least one ASFV protein-encoding polynucleotide selected from the group consisting of the following, but the present invention is not limited thereto:
(a) a Lectin-encoding polynucleotide including the nucleotide sequence represented by SEQ ID NO: 2;
(b) a CD2v-encoding polynucleotide including the nucleotide sequence represented by SEQ ID NO: 4;
(c) a p72-encoding polynucleotide including the nucleotide sequence represented by SEQ ID NO: 6;
(d) a p54-encoding polynucleotide including the nucleotide sequence represented by SEQ ID NO: 8; and
(e) a p30-encoding polynucleotide including the nucleotide sequence represented by SEQ ID NO: 10.

In still another embodiment of the present invention, the recombinant vector may further include at least one selected from the group consisting of the following, but the present invention is not limited thereto:
(a) a polynucleotide encoding NB of SEQ ID NO: 19 or BiP of SEQ ID NO: 21;
(b) a polynucleotide encoding the pFc2 fragment of SEQ ID NO: 27;
(c) a polynucleotide encoding the HDEL peptide of SEQ ID NO: 29; and
(d) a polynucleotide encoding the M domain of SEQ ID NO: 23 or the polyhistidine tag of SEQ ID NO: 25.

In still another embodiment of the present invention, the ASFV antigen protein may be produced in a plant that is transformed with the recombinant vector, but the present invention is not limited thereto.

In still another embodiment of the present invention, the vaccine composition may further include an adjuvant, but the present invention is not limited thereto.

In still another embodiment of the present invention, the adjuvant may be mineral oil or Emulsigen-based, but the present invention is not limited thereto.

In addition, the present invention provides a vaccine kit for preventing African swine fever, including the vaccine composition according to the present invention.

In addition, the present invention provides a method for preventing, ameliorating and/or treating African swine fever, including the step of administering the composition according to the present invention (or a combination of at least one protein selected from the group consisting of Lectin, CD2v, p72, p54 and p30 proteins) to a non-human animal.

Moreover, the present invention provides the use of the composition in the prevention, amelioration or treatment of African swine fever.

Moreover, the present invention provides the use of the composition of claim 1 in the manufacture of a vaccine for preventing African swine fever.

In addition, the present invention provides a feed composition for preventing African swine fever, including a combination of African swine fever virus (ASFV) antigen proteins as an active ingredient, wherein the combination of ASFV antigen proteins is a combination of at least one selected form the group consisting of Lectin, CD2v, p72, p54 and p30 proteins, and wherein when the combination of ASFC antigen proteins includes the Lectin protein and the CD2v protein, the combination of ASFV antigen proteins consists of at least three antigen proteins.

In addition, the present invention provides a recombinant vector for expressing an antigen protein of African swine fever, including a polynucleotide encoding the p72 protein including the amino acid sequence of SEQ ID NO: 5; a polynucleotide encoding the p54 protein including the amino acid sequence of SEQ ID NO: 7; a polynucleotide encoding p15 protein including the amino acid sequence of SEQ ID NO: 11; a polynucleotide encoding the p35 protein including the amino acid sequence of SEQ ID NO: 13; a polynucleotide encoding the E199L protein including the amino acid sequence of SEQ ID NO: 15; or a polynucleotide encoding the F317L protein including the amino acid sequence of SEQ ID NO: 17, wherein the recombinant vector is expressed in a plant.

In an embodiment of the present invention, the recombinant vector may satisfy at least one feature selected from the group consisting of the following, but the present invention is not limited thereto:
(a) the polynucleotide encoding the p72 protein includes the nucleotide sequence represented by SEQ ID NO: 6;
(b) the polynucleotide encoding the p54 protein includes the nucleotide sequence represented by SEQ ID NO: 8;
(c) the polynucleotide encoding the p15 protein includes the nucleotide sequence represented by SEQ ID NO: 12;
(d) the polynucleotide encoding the p35 protein includes the nucleotide sequence represented by SEQ ID NO: 14;
(e) the polynucleotide encoding the E199L protein includes the nucleotide sequence represented by SEQ ID NO: 16; and
(f) the polynucleotide encoding the F317L protein includes the nucleotide sequence represented by SEQ ID NO: 18.

In another embodiment of the present invention, the recombinant vector may further include a polynucleotide encoding the new chaperone binding protein (NB) of SEQ ID NO: 19 or a polynucleotide encoding the chaperone binding protein (BiP) of SEQ ID NO: 21, but the present invention is not limited thereto.

In still another embodiment of the present invention, the polynucleotide encoding the NB or BiP may be located in the 5' terminal direction of the polynucleotide encoding the p72 protein, p54 protein, p15 protein, p35 protein, E199L protein or F317L protein, but the present invention is not limited thereto.

In still another embodiment of the present invention, the recombinant vector may further include a polynucleotide encoding the pFc2 (porcine Fc) fragment of SEQ ID NO: 27, but the present invention is not limited thereto.

In still another embodiment of the present invention, the polynucleotide encoding the pFc2 fragment may be located in the 3' terminal direction of the polynucleotide encoding the p72 protein, p54 protein, p15 protein, p35 protein, E199L protein or F317L protein, but the present invention is not limited thereto.

In still another embodiment of the present invention, the recombinant vector may further include a polynucleotide encoding the HDEL (His-Asp-Glu-Leu) peptide of SEQ ID NO: 29, but the present invention is not limited thereto.

In still another embodiment of the present invention, the polynucleotide encoding the HDEL peptide may be located in the 3' terminal direction of the polynucleotide encoding the p72 protein, p54 protein, p15 protein, p35 protein, E199L protein or F317L protein, but the present invention is not limited thereto.

In still another embodiment of the present invention, the recombinant vector may further include a polynucleotide encoding NB of SEQ ID NO: 19 or BiP of SEQ ID NO: 21; a polynucleotide encoding the pFc2 fragment of SEQ ID NO: 27; and a polynucleotide encoding the HDEL peptide of SEQ ID NO: 29, but the present invention is not limited thereto.

In still another embodiment of the present invention, in the recombinant vector, the polynucleotide encoding NB or BiP; the polynucleotide encoding the p72 protein, p54 protein, p15 protein, p35 protein, E199L protein or F317L protein; the polynucleotide encoding the pFc2 fragment; and the polynucleotide encoding HDEL peptide may be sequentially linked, but the present invention is not limited thereto.

In addition, the present invention provides a transformant, which is transformed with the recombinant vector according to the present invention.

In an embodiment of the present invention, the transformant may be a plant, but the present invention is not limited thereto.

In addition, the present invention provides a method for preparing a recombinant African swine fever virus antigen protein, including the following steps:
(S1) transforming a plant with the recombinant vector according to the present invention; and
(S2) isolating and purifying a recombinant antigen protein from the plant or culture medium.

In an embodiment of the present invention, the NB-encoding polynucleotide may include the nucleotide sequence represented by SEQ ID NO: 20; the BiP-encoding polynucleotide may include the nucleotide sequence represented by SEQ ID NO: 22; the pFc2-encoding polynucleotide may include the nucleotide sequence represented by SEQ ID NO: 28; and the HDEL-encoding polynucleotide may include the nucleotide sequence represented by SEQ ID NO: 30, but the present invention is not limited thereto.

### [Advantageous Effects]

Proteins, especially antigens, for use in the prevention of viral diseases including African swine fever cannot be used in bacteria due to problems such as protein folding and glycosylation, and they are mainly produced by using animal cells. However, for the vaccine production methods using animal cells, the manufacture is not easy because large costs are required to expand facilities for mass production, and antigens are expensive in most cases. In addition, antigens prepared by using animal cells have disadvantages in that they are not easy to store and are highly likely to be contaminated with viruses that are capable of infecting animals. However, the present invention compensated for these disadvantages by using plants. In other words, unlike animal cells, plant cells are very unlikely to be contaminated with viruses that are capable of infecting animals, and mass production is possible at any time as long as the cultivated area is secured, and since long-term storage is possible through plants, the stable and inexpensive production of antigens is possible.

The recombinant African swine fever virus antigen protein of the present invention is not only effectively expressed in plants, but also has high water solubility, and thus, it is easy to isolate and purify the same, and it also acts as an antigen in the body to show high immunogenicity such that it can be used as a novel African swine fever virus vaccine composition. In particular, the inventors of the present invention confirmed that the vaccine composition including the five types of antigen proteins (Lectin, CD2v, p54, p72 and p30) of the present invention is more effective in preventing African swine fever than the vaccine composition that further includes other antigen proteins (p15, p35, E199L, F317L, *etc*.), and thus, it is expected that the recombinant vector and vaccine composition according to the present invention will be widely utilized in the livestock industry.

### [Description of Drawings]

FIG. 1 is a cleavage map showing the sequence of genes for the expression of Lectin, CD2v, p72, p54, p30, p15, p35, E199L and F317L antigen proteins of ASFV in the plant of the present invention.
FIG. 2 shows the results confirmed by Coomassie blue staining after electrophoresis by isolating and purifying the Lectin antigen protein of ASFV.
FIG. 3 shows the results confirmed by Coomassie blue staining after electrophoresis by isolating and purifying the CD2v antigen protein of ASFV.
FIG. 4a is a band photograph obtained by performing Western blot to confirm the expression of the p72 antigen protein of ASFV (DT, Debris Total (total sample before removing debris); T, Total extract (total sample after removing debris); P, Pellet fraction; FT, Flow through fraction, the same below).
FIG. 4b shows the results of Coomassie blue staining after electrophoresis of the protein solution before and after concentration after isolating and purifying the p72 antigen protein of ASFV (left, p72 protein detection result before concentration; right, p72 protein detection result after concentration).
FIG. 5a is a band photograph obtained by Western blotting in order to confirm the expression of the p54 antigen protein of ASFV.
FIG. 5b shows the results obtained by isolating and purifying the ASFV p54 antigen protein, performing electrophoresis of the protein solution before and after concentration, and then confirming by Coomassie blue staining (left, p54 protein detection result before concentration; right, p54 protein detection result after concentration).
FIG. 6 shows the results confirmed by Coomassie blue staining after performing electrophoresis by isolating and purifying the p30 antigen protein of ASFV.
FIG. 7a is a band photograph obtained by Western blotting in order to confirm the expression of the p15 antigen protein of ASFV.
FIG. 7b shows the result of isolating and purifying the p15 antigen protein of ASFV, followed by performing electrophoresis and confirming by Coomassie blue staining.
FIG. 8 shows the results confirmed by Coomassie blue staining after electrophoresis by isolating and purifying the p35 antigen protein of ASFV.
FIG. 9a is a band photograph obtained by performing Western blotting in order to confirm the expression of the E199L antigen protein of ASFV.
FIG. 9b shows the results obtained by isolating and purifying the ASFV E199L antigen protein, performing electrophoresis of the protein solution before and after concentration, and then confirming by Coomassie blue staining (left, E199L protein detection result before concentration; right, E199L protein detection result after concentration).
FIG. 10a is a band photograph obtained by performing Western blot in order to confirm the expression of the F317L antigen protein of ASFV extracted from 100 g or 1 kg of *Nicotiana bentamia* leaves (top, F317L protein detection result extracted from 100 g of leaves; Bottom, F317L protein detection result extracted from 1 kg of leaves).
FIG. 10b shows the results of isolating and purifying the F317L protein of ASFV extracted from 100 g or 1 kg of *Nicotiana bentamia* leaves, performing electrophoresis and confirming by Coomassie blue staining (Left, F317L protein detection result extracted from 100 g of leaves; Right, F317L protein detection result extracted from 1 kg of leaves).
FIG. 11 is the results of confirming the body temperature and survival rate of pigs over time by conducting a challenge inoculation experiment after treating pigs with a 5-antigen vaccine (combination of Lectin, CD2v, p54, p72 and p30), a 9-antigen vaccine (combination of Lectin, CD2v, p54, p72, p30, p15, p35, E199L and F317L), or PBS according to the present invention.
FIG. 12 is the results of measuring the level of viruses (Viremia) in the blood of pigs over time by conducting a challenge inoculation experiment after treating pigs with the 5-antigen vaccine, the 9-antigen vaccine or PBS according to the present invention.

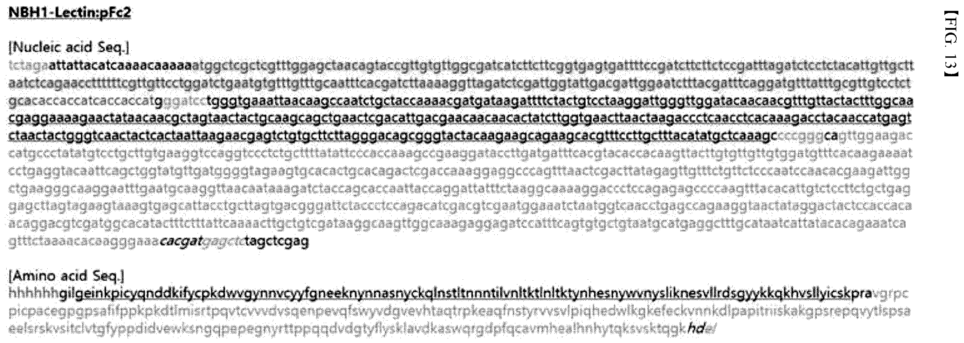

FIG. 13 shows a gene sequence (top) and an amino acid sequence (bottom) for expressing the Lectin recombinant protein according to an embodiment of the present invention.

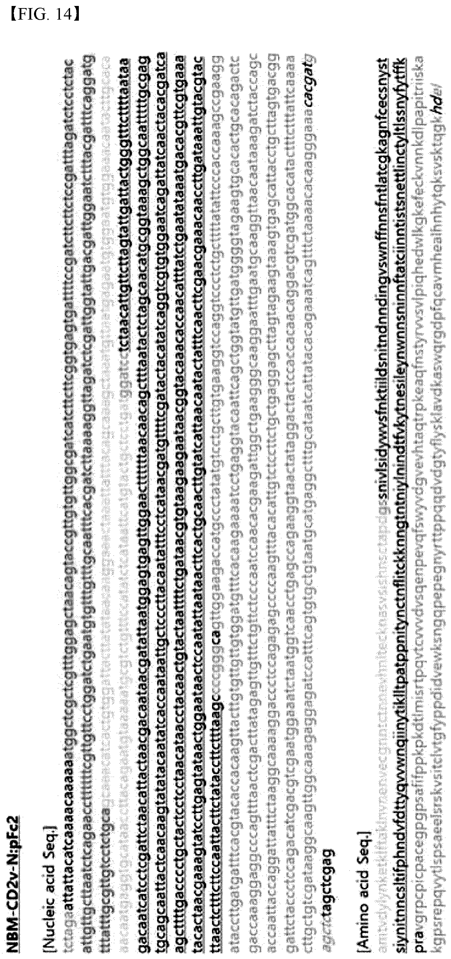

FIG. 14 shows a gene sequence (top) and an amino acid sequence (bottom) for expressing the CD2v recombinant protein according to an embodiment of the present invention.

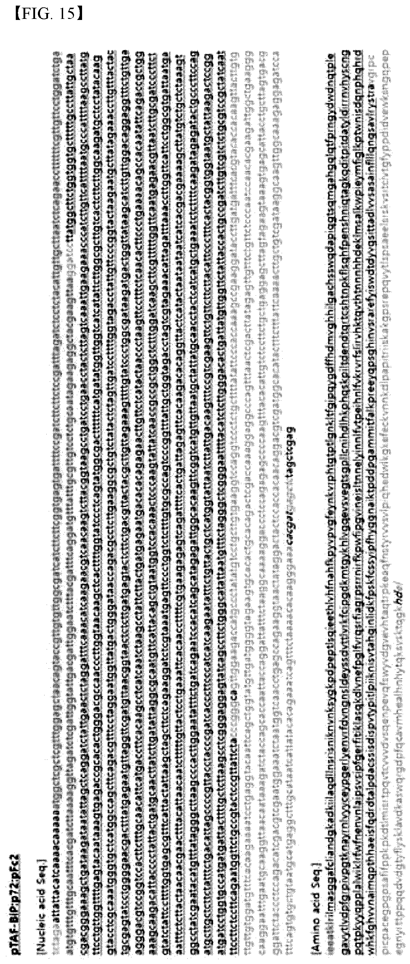

FIG. 15 shows a gene sequence (top) and an amino acid sequence (bottom) for expressing the p72 recombinant protein according to an embodiment of the present invention.

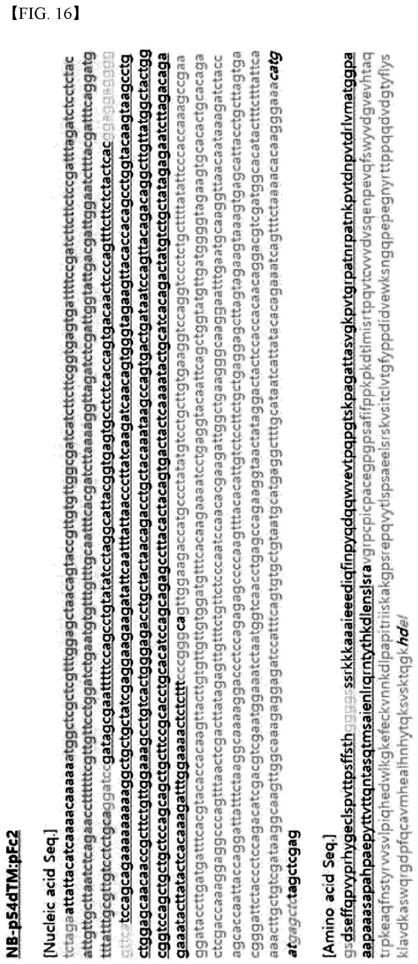

FIG. 16 shows a gene sequence (top) and an amino acid sequence (bottom) for expressing the p54 recombinant protein according to an embodiment of the present invention.

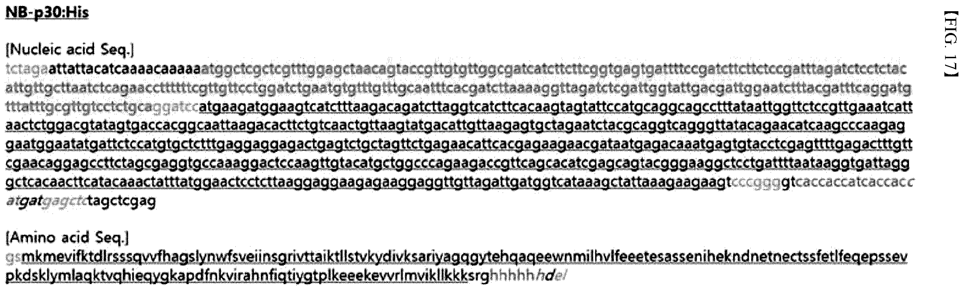

FIG. 17 shows a gene sequence (top) and an amino acid sequence (bottom) for expressing the p30 recombinant protein according to an embodiment of the present invention.

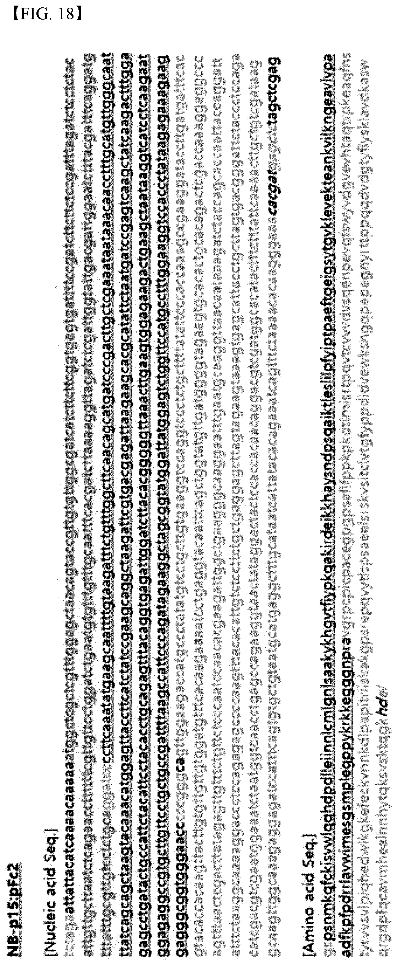

FIG. 18 shows a gene sequence (top) and an amino acid sequence (bottom) for expressing the p15 recombinant protein expression according to an embodiment of the present invention.

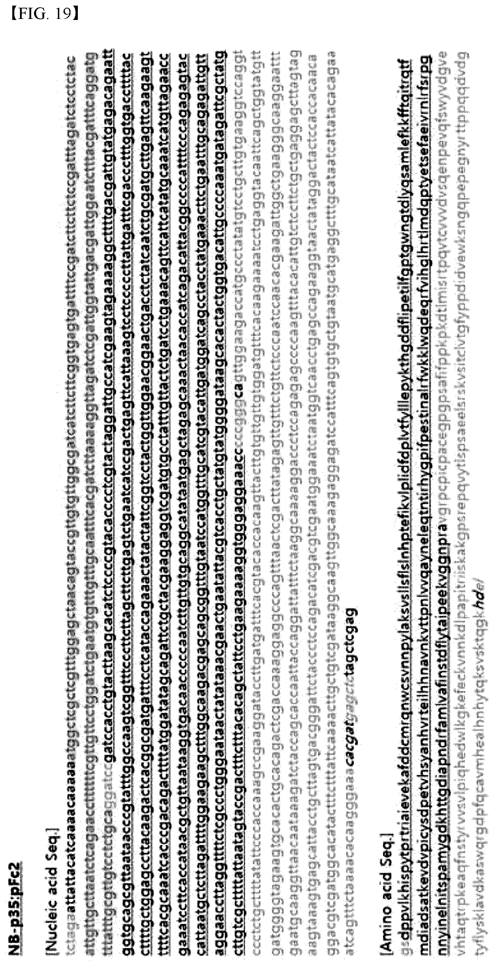

FIG. 19 shows a gene sequence (top) and an amino acid sequence (bottom) for expressing the p35 recombinant protein according to an embodiment of the present invention.

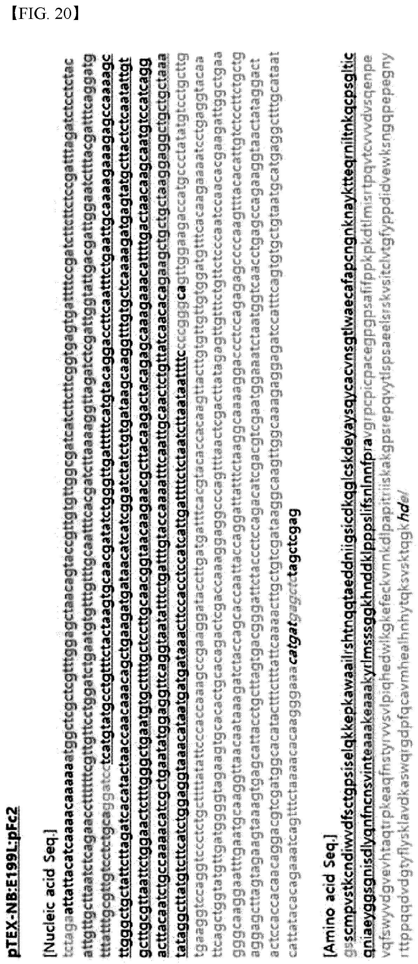

FIG. 20 shows a gene sequence (top) and an amino acid sequence (bottom) for expressing the E199L recombinant protein according to an embodiment of the present invention.

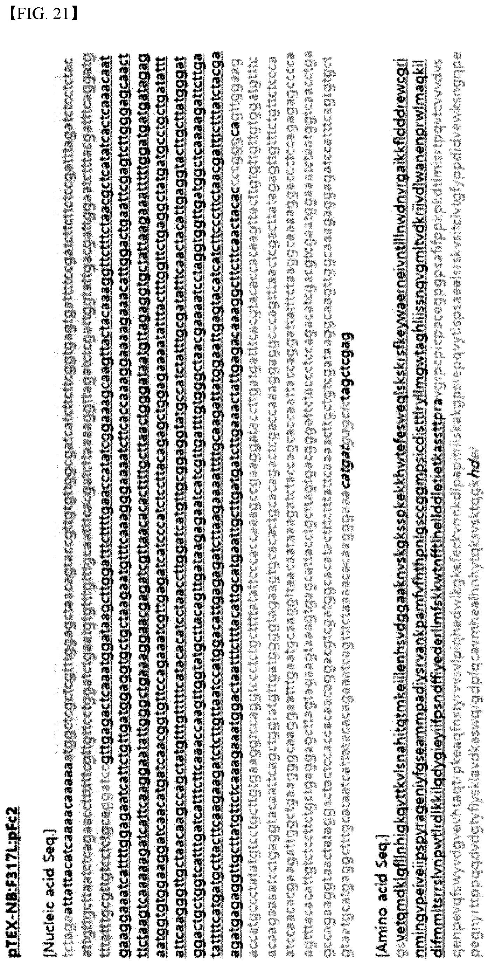

FIG. 21 shows a gene sequence (top) and an amino acid sequence (bottom) for expressing the F317L recombinant protein according to an embodiment of the present invention.
FIG. 22 shows the results of measuring the level of anti-p30 antibody on days 0, 7, 14, 21, 28 and 35, after administering a 5-antigen vaccine (combination of Lectin, CD2v, p54, p72 and p30; "Vax grp1"), a 9-antigen vaccine (combination of Lectin, CD2v, p54, p72, p30, p15, p35, E199L and F317L) or PBS ("PBS") according to the present invention to pigs.

### [Modes of the Invention]

Unless defined otherwise, all technical and scientific terms used in the present specification have the same meaning as commonly understood by one of ordinary skill in the art to which this invention pertains. In general, the nomenclature used in the present specification is the nomenclature that is well known and commonly used in the art.

The inventors of the present invention confirmed that by using the genes of the 5 types of antigen proteins Lectin, CD2v, p72, p54, p30, p15, p35, E199L and F317L of African swine fever virus (ASFV), each of the above ASFV antigen proteins with high immunogenicity can be efficiently produced and isolated in plants. Therefore, since the African swine fever virus antigen proteins of the present invention can be stably and efficiently mass-produced, it is possible to provide an inexpensive and stable African swine fever virus vaccine. In addition, the inventors of the present invention confirmed that when a vaccine composition including five antigen proteins, including Lectin, CD2v, p72, p54 and p30, was administered to pigs, it exerted an excellent effect of preventing African swine fever, and the effect was more remarkable compared to a vaccine composition further including other ASFV antigen proteins such as p15, p35, E199L and F317L. That is, the present invention is significant in discovering the optimal antigen protein combination (Lectin, CD2v, p72, p54 and p30) that can achieve a more significant effect of preventing African swine fever than the prior art.

Accordingly, an object of the present invention is to provide a recombinant vector for expressing an ASFV antigen protein including a combination of African swine fever virus (ASFV) protein-encoding polynucleotides, and the combination of the ASFV protein-encoding polynucleotides is characterized in that it is a combination of at least one selected from the group consisting of:
a polynucleotide encoding Lectin protein including the amino acid sequence of SEQ ID NO: 1;
a polynucleotide encoding CD2v protein including the amino acid sequence of SEQ ID NO: 3;
a polynucleotide encoding p72 protein including the amino acid sequence of SEQ ID NO: 5;
a polynucleotide encoding p54 protein including the amino acid sequence of SEQ ID NO: 7;
a polynucleotide encoding p30 protein including the amino acid sequence of SEQ ID NO: 9;
a polynucleotide encoding p15 protein including the amino acid sequence of SEQ ID NO: 11;
a polynucleotide encoding p35 protein including the amino acid sequence of SEQ ID NO: 13;
a polynucleotide encoding E199L protein including the amino acid sequence of SEQ ID NO: 15; and
a polynucleotide encoding F317L protein including the amino acid sequence of SEQ ID NO: 17.

More preferably, an object of the present invention is to provide a recombinant vector for expressing an ASFV antigen protein including a combination of African swine fever virus (ASFV) protein-encoding polynucleotides, and the combination of the ASFV protein-encoding polynucleotides is characterized in that it is a combination of at least one selected from the group consisting of:
a polynucleotide encoding Lectin protein including the amino acid sequence of SEQ ID NO: 1;
a polynucleotide encoding CD2v protein including the amino acid sequence of SEQ ID NO: 3;
a polynucleotide encoding p72 protein including the amino acid sequence of SEQ ID NO: 5;
a polynucleotide encoding p54 protein including the amino acid sequence of SEQ ID NO: 7; and
a polynucleotide encoding p30 protein including the amino acid sequence of SEQ ID NO: 9.

The order in which each of the polynucleotides is arranged in the recombinant vector is not limited.

As used herein, the "African swine fever virus" is a DNA virus of about 200 nm belonging to the family *Asfarviridae* and the genus *Asfivirus,* and it is the causative factor of African swine fever (ASF). The ASFV continuously infects its natural hosts, pigs, warthogs, wild boars and the genus *Ornithodoros* belonging to the family *Argasidae,* and it causes fatal hemorrhagic fever upon infection.

Preferably, the recombinant vector according to the present invention includes the above five ASFV protein (Lectin, CD2v, p72, p54 and p30) coding-polynucleotides, and it is characterized by not including other ASFV protein-coding polynucleotides. More preferably, the recombinant vector according to the present invention is characterized in that it does not include a polynucleotide encoding the ASFV antigen protein p15, p35, E199L or F317L.

The Lectin antigen protein may include the amino acid sequence of SEQ ID NO: 1 or be encoded by a polynucleotide including the nucleotide sequence of SEQ ID NO: 2, and most preferably, it may consist of the amino acid sequence of SEQ ID NO: 1 or be encoded by a polynucleotide consisting of the nucleotide sequence of SEQ ID NO: 2, but the present invention is not limited thereto. Preferably, the Lectin protein according to the present invention may include a transmembrane region, or may be a Lectin protein from which the transmembrane region has been removed.

Specifically, the polynucleotide encoding the Lectin protein may consist of the nucleotide sequence represented by SEQ ID NO: 2, but the present invention is not limited thereto, and variants of the nucleotide sequence are included within the scope of the present invention. The nucleic acid molecule of the nucleotide sequence represented by SEQ ID NO: 2 of the present invention is a functional equivalent of the nucleic acid molecule constituting the same, and for example, it is a concept that includes variants that can have the same functional effect as the nucleic acid molecule, although some nucleotide sequences of a nucleic acid molecule have been modified by deletion, substitution or insertion. Specifically, the polynucleotide encoding the Lectin protein may have a nucleotide sequence having sequence homology of at least 70%, more preferably, at least 80%, even more preferably, at least 90%, and most preferably, at least 95% to the nucleotide sequence represented by SEQ ID NO: 2, respectively. For example, it includes a nucleotide sequence having sequence homology of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. The "% of sequence homology" for a polynucleotide is determined by comparing a comparison region with two optimally aligned sequences, and in the comparison region, parts of a polynucleotide sequence may include additions or deletions (i.e., gaps) compared to a reference sequence (which does not include additions or deletions) for the optimal alignment of the two sequences.

In addition, the CD2v antigen protein may include the amino acid sequence of SEQ ID NO: 3, may be encoded by a polynucleotide including the nucleotide sequence of SEQ ID NO: 4, and may most preferably consist of the amino acid sequence of SEQ ID NO: 3 or SEQ ID NO: 4, or may be encoded by a polynucleotide consisting of the nucleotide sequence of SEQ ID NO: 4, but the present invention is not limited thereto. That is, the polynucleotide encoding the CD2v protein may include a nucleotide sequence having sequence homology of at least 70%, more preferably, at least 80%, still more preferably, at least 90%, and most preferably, at least 95% to the nucleotide sequence represented by SEQ ID NO: 4, respectively. Preferably, CD2v according to the present invention may include the transmembrane domain (TMD) of the CD2v protein, or may be a CD2v protein in which the transmembrane domain is removed, and preferably, it may be the N-terminal portion from the transmembrane domain of the CD2v protein.

In addition, the p72 antigen protein may include the amino acid sequence of SEQ ID NO: 5, may be encoded by a polynucleotide including the nucleotide sequence of SEQ ID NO: 6, may most preferably consist of the amino acid sequence of SEQ ID NO: 5, or may be encoded by a polynucleotide consisting of the nucleotide sequence of SEQ ID NO: 6, but the present invention is not limited thereto. That is, the polynucleotide encoding the p72 protein may include a nucleotide sequence having sequence homology of at least 70%, more preferably, at least 80%, still more preferably, at least 90%, and most preferably, at least 95% to the nucleotide sequence represented by SEQ ID NO: 6, respectively.

In addition, the p54 antigen protein may include the amino acid sequence of SEQ ID NO: 7, may be encoded by a polynucleotide including the nucleotide sequence of SEQ ID NO: 8, may most preferably consist of the amino acid sequence of SEQ ID NO: 7, or may be encoded by a polynucleotide consisting of the nucleotide sequence of SEQ ID NO: 8, but the present invention is not limited thereto. That is, the polynucleotide encoding the p54 protein may include a nucleotide sequence having sequence homology of at least 70%, more preferably, at least 80%, still more preferably, at least 90%, and most preferably, at least 95% to the nucleotide sequence represented by SEQ ID NO: 8, respectively. Preferably, the p54 protein according to the present invention may be a p54 protein (p54dTM) in which the transmembrane region is removed. More preferably, the p54 protein according to the present invention may include a transmembrane region. Alternatively, the p54 protein may have a transmembrane region thereof removed and a linker sequence inserted in the place thereof. The linker sequence may be Gly-Gly-Gly-Gly-Ser.

In addition, the p30 antigen protein may include the amino acid sequence of SEQ ID NO: 9, may be encoded by a polynucleotide including the nucleotide sequence of SEQ ID NO: 10, may most preferably consist of the amino acid sequence of SEQ ID NO: 9, or may be encoded by a polynucleotide consisting of the nucleotide sequence of SEQ ID NO: 10, but the present invention is not limited thereto. That is, the polynucleotide encoding the p30 protein may include a nucleotide sequence having sequence homology of at least 70%, more preferably, at least 80%, still more preferably, at least 90%, and most preferably, at least 95% to the nucleotide sequence represented by SEQ ID NO: 10, respectively.

In addition, the p15, p35, E199L and F317L antigen proteins may respectively include, in order, the amino acid sequence of SEQ ID NO: 11, SEQ ID NO: 13, SEQ ID NO: 15 or SEQ ID NO: 17, may be respectively encoded by a polynucleotide including the nucleotide sequence of SEQ ID NO: 12, SEQ ID NO: 14, SEQ ID NO: 16 or SEQ ID NO: 18, may most preferably respectively consist of the amino acid sequence of SEQ ID NO: 11, SEQ ID NO: 13, SEQ ID NO: 15 or SEQ ID NO: 17, or may respectively encoded by a polynucleotide consisting of the nucleotide sequence of SEQ ID NO: 12, SEQ ID NO: 14, SEQ ID NO: 16 or SEQ ID NO: 18, but the present invention is not limited thereto. That is, the polynucleotide encoding the p15, p35, E199L or F317L antigen protein may respectively include, in order, a nucleotide sequence having sequence homology of at least 70%, more preferably, at least 80%, still more preferably, at least 90%, and most preferably, at least 95% to the nucleotide sequence represented by SEQ ID NO: 12, SEQ ID NO: 14, SEQ ID NO: 16 or SEQ ID NO: 18, respectively.

In addition, the E199L may include a transmembrane region or may exclude a transmembrane region.

In the present specification, a polypeptide (or peptide) consisting of an amino acid sequence represented by a specific sequence number is a concept including variants that can functionally perform the same function as a polypeptide molecule, although a functional equivalent of the polypeptide molecule constituting the same, for example, some of the amino acid sequences have been modified by deletion, substitution or insertion. Specifically, the polypeptide represented by a specific sequence number may include an amino acid sequence having sequence homology of at least 70%, more preferably, at least 80%, still more preferably, at least 90%, and most preferably, at least 95% to the amino acid sequence represented by the corresponding sequence number. For example, it includes an amino acid sequence having sequence homology of 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% or 100%. The "% of sequence homology" for an amino acid sequence is determined by comparing a comparison region with two optimally aligned sequences, and in the comparison region, parts of an amino acid sequence may include additions or deletions (i.e., gaps) compared to a reference sequence (which does not include additions or deletions) for the optimal alignment of the two sequences.

The Lectin, CD2v, p54, p72, p30, p15, p35, E199L and F317L antigen proteins are antigen proteins of African swine fever virus, and may regulate the immune response mechanism of a host.

As used herein, the term "polynucleotide" refers to an oligomer or polymer including two or more linked nucleotides or nucleotide derivatives that are generally bound to each other via a phosphodiester bond, including deoxyribonucleic acid (DNA) and ribonucleic acid (RNA). Polynucleotides also include, for example, nucleotide analogues, or DNA and RNA derivatives including "skeletal" bonds other than phosphodiester bonds such as phosphotriester bonds, phosphoramidate bonds, phosphorothioate bonds, thioester bonds or peptide bonds (peptide nucleic acids). Polynucleotides include single-stranded and/or double-stranded polynucleotides, such as deoxyribonucleic acid (DNA) and ribonucleic acid (RNA), as well as analogues of either RNA or DNA.

As used herein, the term "antigen" refers to all substances that cause an immune response in the body, and preferably may be viruses, chemicals, bacteria, pollen, cancer cells or the like, or some peptides or proteins thereof, but the present invention is not limited thereto as long as it is a substance that can cause an immune response in the body.

In an embodiment of the present invention, the recombinant vector may further include a polynucleotide encoding a new chaperone binding protein (NB) or a chaperone binding protein (BiP) signal peptide, a polynucleotide encoding porcine Fc fragments, a polypeptide encoding an HDEL (His-Asp-Glu-Leu) peptide, a polynucleotide encoding an M domain, and/or a polynucleotide encoding a polyhistidine tag.

In another embodiment of the invention, the recombinant vector may satisfy at least one feature of the following:
(a) the polynucleotide encoding the NB or BiP is located in the 5' terminal direction of the polynucleotide encoding the ASFV antigen protein;
(b) the polynucleotide encoding the pFc2 fragment is located in the 3' terminal direction of the polynucleotide encoding the ASFV antigen protein;
(c) the polynucleotide encoding the HDEL peptide is located in the 3' terminal direction of the polynucleotide encoding the ASFV antigen protein; or
(d) the polynucleotide encoding the M domain or polyhistidine tag is located in the 5' terminal or 3' terminal direction of the polynucleotide encoding the ASFV antigen protein.

Preferably, the recombinant vector may include all of a polynucleotide encoding the NB or BiP; a polynucleotide encoding the pFc2 fragment; and a polynucleotide encoding the HDEL peptide, and in this case, the order of linkage of each gene is not limited, but preferably, in the recombinant vector, a polynucleotide encoding NB or BiP; polynucleotides encoding Lectin, CD2v, p72, p54 and/or p30 proteins; a polynucleotide encoding the pFc2 fragment; and a polynucleotide encoding the HDEL peptide may be sequentially linked.

Alternatively, the recombinant vector may include all of a polynucleotide encoding the NB or BiP; a polynucleotide encoding the pFc2 fragment; and a polynucleotide encoding the HDEL peptide, and in this case, the order of linkage of each gene is not limited, but preferably, in the recombinant vector, a polynucleotide encoding NB or BiP; polynucleotides encoding Lectin, CD2v, p72, p54, p30, p15, p35, E199L and/or F317L proteins; a polynucleotide encoding the pFc2 fragment; and a polynucleotide encoding the HDEL peptide may be sequentially linked.

Most preferably, the recombinant vector may include all of a polynucleotide encoding the NB or BiP; a polynucleotide encoding the pFc2 fragment; a polynucleotide encoding the HDEL peptide; and a polynucleotide encoding an M domain or a polyhistidine tag, and in this case, the order of linkage of each gene is not limited, but preferably, in the recombinant vector, a polynucleotide encoding NB or BiP; M domain or polyhistidine tag; polynucleotides encoding Lectin, CD2v, p72, p54 and/or p30 proteins; a polynucleotide encoding the pFc2 fragment; and a polynucleotide encoding the HDEL peptide may be sequentially linked, or a polynucleotide encoding NB or BiP; polynucleotides encoding Lectin, CD2v, p72, p54 and/or p30 proteins; M domain or polyhistidine tag; a polynucleotide encoding the pFc2 fragment; and a polynucleotide encoding the HDEL peptide may be sequentially linked.

Alternatively, the recombinant vector may include all of a polynucleotide encoding the NB or BiP; a polynucleotide encoding the pFc2 fragment; a polynucleotide encoding the HDEL peptide; and a polynucleotide encoding an M domain or a polyhistidine tag, and in this case, the order of linkage of each gene is not limited, but preferably, in the recombinant vector, a polynucleotide encoding NB or BiP; M domain or polyhistidine tag; polynucleotides encoding Lectin, CD2v, p72, p54, p30, p15, p35, E199L and/or F317L proteins; a polynucleotide encoding the pFc2 fragment; and a polynucleotide encoding the HDEL peptide may be sequentially linked, or a polynucleotide encoding NB or BiP; polynucleotides encoding Lectin, CD2v, p72, p54, p30, p15 protein, p35 protein, E199L protein and/or F317L protein; M domain or polyhistidine tag; a polynucleotide encoding the pFc2 fragment; and a polynucleotide encoding the HDEL peptide may be sequentially linked.

In another embodiment of the present invention, the polynucleotide encoding the polyhistidine tag may be characterized in that it is located only in the 5' terminal direction of the polynucleotide encoding Lectin and/or in the 3' terminal direction of the polynucleotide encoding p30 among the ASFV antigen protein genes, and the M domain may be characterized in that it is located only in the 5' terminal direction of the polynucleotide encoding CD2v among the ASFV antigen protein genes. In still another embodiment, the polynucleotide encoding the pFc2 fragment may be characterized in that it is not located in the 3' terminal direction of the polynucleotide encoding p30.

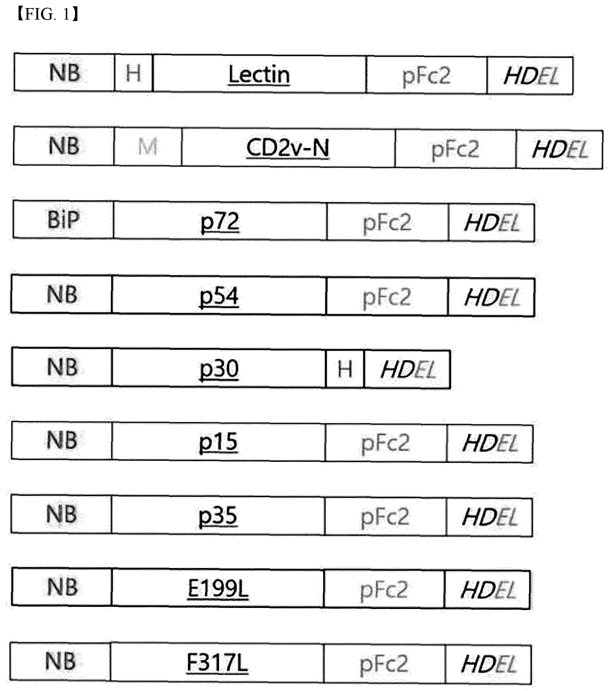

When linked in the same order as above, that is, when the recombinant vector includes the expression cassette shown in the cleavage map of FIG. 1, the recombinant vector according to the present invention may include the nucleotide sequence represented by SEQ ID NO: 32, SEQ ID NO: 34, SEQ ID NO: 36, SEQ ID NO: 38, SEQ ID NO: 40, SEQ ID NO: 47, SEQ ID NO: 48, SEQ ID NO: 49 or SEQ ID NO: 50, and may most preferably consist of the nucleotide sequence represented by SEQ ID NO: 32, SEQ ID NO: 34, SEQ ID NO: 36, SEQ ID NO: 38, SEQ ID NO: 40, SEQ ID NO: 47, SEQ ID NO: 48, SEQ ID NO: 49 or SEQ ID NO: 50, or it may include a nucleotide sequence having sequence homology of at least 80%, more preferably, at least 90%, and still more preferably, at least 95% to the nucleotide sequence represented by SEQ ID NO: 32, SEQ ID NO: 34, SEQ ID NO: 36, SEQ ID NO: 38, SEQ ID NO: 40, SEQ ID NO: 47, SEQ ID NO: 48, SEQ ID NO: 49,or SEQ ID NO: 50. The nucleotide sequence represented by SEQ ID NO: 32, SEQ ID NO: 34, SEQ ID NO: 36, SEQ ID NO: 38, SEQ ID NO: 40, SEQ ID NO: 47, SEQ ID NO: 48, SEQ ID NO: 49 or SEQ ID NO: 50 may respectively be included in plasmid vectors that are independent of each other, and a combination of at least one selected from the group consisting of SEQ ID NO: 32, SEQ ID NO: 34, SEQ ID NO: 36, SEQ ID NO: 38, SEQ ID NO: 40, SEQ ID NO: 47, SEQ ID NO: 48, SEQ ID NO: 49 and SEQ ID NO: 50 may be included in a single plasmid vector.

As used herein, the term "recombinant vector" refers to a vector which is capable of expressing a peptide or protein encoded by a heterologous nucleic acid inserted into the vector, and preferably means a vector constructed so as to express a target antigen (in the present invention, ASFV Lectin, CD2v, p54, p72, p30, p15, p35, E199L and/or F317L). The term "vector" as used herein refers to any vehicle for the introduction and/or transfer of a nucleotide into a host cell *in vitro, ex vivo* or *in vivo,* and may mean a replicon to which another DNA fragment may be attached so as to bring about the replication of the attached fragment capable of binding another DNA fragment to bring about replication of the bound fragment (replicon), and the term "replicon" refers to any genetic unit (*e.g.,* a plasmid, a phage, a cosmid, a chromosome, a virus, *etc.*) that functions as an autonomous unit of DNA replication *in vivo,* that is, it is capable of replicating by self-regulation. Preferably, the recombinant vector according to the present invention may be characterized in that it is expressed in a plant.

The recombinant vector of the present invention may include, preferably, a promoter which is a transcription initiation factor to which an RNA polymerase binds, an arbitrary operator sequence for regulating transcription, a sequence encoding a suitable mRNA ribosome binding site, a sequence regulating the termination of transcription and translation, a terminator or the like, and more preferably, it may further include a polyhistidine tag (an amino acid motif composed of at least 5 or more histidine residues), an endoplasmic reticulum signal peptide (meaning the same sequence as an endoplasmic reticulum targeting sequence) gene, an endoplasmic reticulum retention signal peptide, a cloning site or the like, and more preferably, it may further include marker genes for selection such as additional tag genes and antibiotic resistance genes for selecting transformants, in addition to the Fc fragment, which is a tag.

Examples of the gene for a tag may representatively include an Avi tag, a Calmodulin tag, a polyglutamate tag, an E tag, a FLAG tag, an HA tag, a His tag (polyhistidine tag), an Myc tag, an S tag, an SBP tag, an IgG-Fc tag, a CTB. tag, a Softag 1 tag, a Softag 3 tag, a Strep tag, a TC tag, a V5 tag, a VSV tag, an Xpress tag and the like.

As used herein, the term "Fc fragment" refers to a portion which is linked only to a heavy chain (H chain) portion by an S-S bond and does not have any antigen binding site when the immunoglobulin is digested by papain, and the Fc fragment of the present invention is preferably a porcine Fc fragment, and more preferably, a porcine Fc fragment (pFc2) represented by SEQ ID NO: 28, but the present invention is not limited thereto. In addition, variants of the nucleotide sequence represented by SEQ ID NO: 28 of the Fc fragment of the present invention are included in the scope of the present invention. Specifically, the gene may include a nucleotide sequence having sequence homology of at least 90%, more preferably, at least 95%, and most preferably, at least 98% to the nucleotide sequence of SEQ ID NO: 28.

The term "cloning site" is a general term for those inserted for the purpose of linking/isolating each gene in a vector. Preferably, the cloning site may be a sequence represented by "tctaga", a sequence represented by "ggatcc", a sequence represented by "cccggg", or a sequence represented by "gagctc" in SEQ ID NOs: 2, 4, 6, 8 and 10, but the present invention is not limited thereto.

The "ER signal peptide" is a signal peptide located at the N-terminus of a protein, and serves to induce the newly synthesized protein to enter the endoplasmic reticulum (ER). The type and amino acid sequence of the endoplasmic reticulum signal peptide according to the present invention are not limited as long as it is a plant endoplasmic reticulum signal peptide known to those skilled in the art, but it may be preferably selected from new chaperone binding protein (NB) and chaperone binding protein (BiP).

The "NB (new chaperone binding protein) gene" is preferably a gene including the nucleotide sequence of SEQ ID NO: 20, and most preferably, it may be a gene represented by SEQ ID NO: 20, or include a nucleotide sequence having sequence homology of at least 80%, more preferably, at least 90%, and still more preferably, at least 95% to the nucleotide sequence of SEQ ID NO: 20. In addition, the "BiP (chaperone binding protein) gene" is preferably a gene including the nucleotide sequence of SEQ ID NO: 22, and most preferably, it may be a gene represented by SEQ ID NO: 22, or include a nucleotide sequence having sequence homology of at least 80%, more preferably, at least 90%, and still more preferably, at least 95% to the nucleotide sequence of SEQ ID NO: 22. As described above, the NB or BiP gene is used to transfer the expressed recombinant protein to the endoplasmic reticulum, and when the recombinant protein is expressed, a part of the sequence may be truncated and only some amino acids may remain, or the entire sequence may be truncated such that the signal peptide sequence region does not exist.

The "ER retention signal peptide" is a signal peptide which is located at the C-terminus of a protein, and when a protein present in the endoplasmic reticulum escapes to the Golgi apparatus through the secretory pathway, it plays a role in allowing the protein to return to the endoplasmic reticulum (retention). The type and amino acid sequence of the endoplasmic reticulum residual signal peptide according to the present invention are not limited as long as it is a plant residual endoplasmic reticulum signal peptide known to those skilled in the art, but it may be preferably selected from KDEL (Lys-Asp-Glu-Leu) sequence and HDEL sequence.

Most preferably, the amino acid sequence of the ER residual signal peptide may be HDEL (His-Asp-Glu-Leu, amino acid represented by SEQ ID NO: 29), or may be encoded by the nucleotide sequence represented by SEQ ID NO: 30. In addition, the endoplasmic reticulum signal peptide of the present invention includes a variant of SEQ ID NO: 30 within the scope of the present invention. Specifically, the gene may include a nucleotide sequence having sequence homology of at least 90%, more preferably, at least 95%, and most preferably, at least 98% to the nucleotide sequence of SEQ ID NO: 30. The binding site of the endoplasmic reticulum signal peptide is characterized in that it is added to (or linked to) the C-terminus of a protein intended for expression or synthesis in a plant cell.

For information on the endoplasmic reticulum signal peptide and the endoplasmic reticulum residual signal peptide, reference may be made to documents such as US 20130295065 and WO2009158716.

Examples of the selection marker gene may include a herbicide-resistant gene such as glyphosate or phosphinothricin, an antibiotic-resistant gene such as kanamycin, G418, bleomycin, hygromycin or chloramphenicol, or an aadA gene, and examples of the promoter may include a pEMU promoter, a MAS promoter, a histone promoter, a Clp promoter, a cauliflower mosaic virus-derived 35S promoter, a cauliflower mosaic virus-derived 19S RNA promoter, a plant actin protein promoter, a ubiqitin protein promoter, a cytomegalovirus (CMV) promoter, a simian virus 40 (SV40) promoter, a respiratory syncytial virus (RSV) promoter, an elongation factor-1 alpha (EF-1α) promoter, a pEMU promoter, a MAS promoter, a histone promoter, a Clp promoter, a MacT (CaMV 35S+MAS; 3' terminal nucleotide of Mac promoter is replaced with T) promoter and the like, and examples of the terminator may include a nopaline synthase (NOS) terminator, a rice amylase RAmy1 A terminator, a faceolin terminator, a terminator of an octopine gene of *Agrobacterium tumafaciens,* an rmB1/B2 terminator of E. *coli,* a HSP18.2 terminator of *Arabidopsis thaliana,* an RD29B terminator of *Arabidopsis thaliana,* but those listed above are examples, and the present invention is not limited thereto.

In another aspect of the present invention, the present invention provides a transformant which is transformed with the recombinant vector.

In an embodiment of the present invention, the transformant is preferably a microorganism such as *E. coli, Bacillus, Salmonella* or a yeast, insect cells, animal cells including human cells, an animal such as a mouse, a rat, a dog, a monkey, a pig, a horse or a cow, *Agrobacterium tumefaciens,* or a plant, and more preferably, a food crop such as rice, wheat, barley, corn, bean, potato, red bean, oat or sorghum; a vegetable crop such as *Arabidopsis,* Chinese cabbage, radish, red pepper, strawberry, tomato, watermelon, cucumber, cabbage, oriental melon, pumpkin, green onion, onion or carrot; a specialty crop such as ginseng, tobacco, cotton, sesame, sugar cane, sugar beet, perilla, peanut or rapeseed; a fruit tree such as an apple tree, a pear tree, a date tree, peach, grape, tangerine, persimmon, plum, apricot or banana; and a flower such as a rose, a carnation, a chrysanthemum, lily or tulip, but as long as the transformant is an organism that can be transformed with the vector of the present invention, the present invention is not limited thereto. Most preferably, the transformant may be a plant of the genus *Nicotiana.*

As used herein, the term "transformation" encompasses a change in genetic properties of a living organism by injected DNA, and the term "transgenic organism" is a living organism produced by injecting an external gene through the molecular genetic method, and preferably, a living organism transformed by a recombinant expression vector of the present invention. The living organism is a living thing such as a microorganism, a eukaryotic cell, an insect, an animal or a plant without limitation, and preferably, *E. coli, Salmonella, Bacillus,* yeast, an animal cell, a mouse, a rat, a dog, a monkey, a pig, a horse, a cow, *Agrobacterium tumefaciens* or a plant, but the present invention is not limited thereto.

As used herein, the term "plant" is a plant that can produce a protein in a large amount, and more specifically, it may be selected from the group consisting of tobacco, *Arabidopsis thaliana,* corn, rice, soybean, canola, alfalfa, sunflower, sorghum, wheat, cotton, peanut, tomato, potato, lettuce and pepper, and preferably tobacco. The tobacco in the present invention is a *Nicotiana* genus plant, which can overexpress a protein, but there is no particular limitation on the type of tobacco, and the present invention may be implemented by selecting a suitable species for a transforming method and the purpose of mass-production of a protein. For example, a species such as *Nicotiana benthamiana* L. or *Nicotiana tabacum cv. Xanthi* may be used.

The transformant may be prepared by transformation, transfection, *Agrobacterium-mediated* transformation, particle gun bombardment, sonication, electroporation and polyethylene glycol (PEG)-mediated transformation, but there is no limitation as long as the transformant is prepared by any method of injecting the vector of the present invention.

In another aspect of the present invention, the present invention provides a recombinant protein for inducing antibodies against African swine fever virus, which is produced by using the recombinant vector according to the present invention. That is, the present invention provides Lectin, CD2v, p54, p72, p30, p15, p35, E199L and F317L recombinant antigen proteins that are produced by using the recombinant vector according to the present invention. The antigen proteins may be produced from separate recombinant vectors (i.e., each separate nucleic acid molecule), or may be produced from a recombinant vector including polynucleotides encoding two or more recombinant proteins (*i.e.,* a single nucleic acid molecule). In particular, the main object of the present invention is to provide Lectin, CD2v, p54, p72 and p30 recombinant antigen proteins that are produced by using the recombinant vector according to the present invention.

In an embodiment of the present invention, the Lectin, CD2v, p54, p72, p30, p15, p35, E199L and F317L recombinant antigen proteins may be water-soluble. More specifically, 60%, 65%, 70%, 75%, 80%, 85%, 90%, 91%, 92%, 93%, 94%, 95%, 96%, 97%, 98%, 99% or 100% of Lectin, CD2v, p54, p72, p30, p15, p35, E199L and F317L recombinant antigen proteins that are expressed in plants may be dissolved in a water-soluble fraction.

In another embodiment of the present invention, the Lectin, CD2v, p54, p72, p30, p15, p35, E199L and F317L recombinant antigen proteins may be isolated and purified with a purity of 85% or more. More specifically, when Lectin, CD2v, p54, p72, p30, p15, p35, E199L and F317L recombinant antigen proteins that are expressed in plants by using the recombinant vector according to the present invention are isolated and purified by using conventional methods, it is possible to obtain Lectin, CD2v, p54, p72, p30, p15, p35, E199L and F317L recombinant antigen proteins at a purity of 85%, 86%, 87%, 88%, 89%, 90%, 91%, 92%, 93%, 94%, 95%, 96%, 97%, 98%, 99% or 100%.

In another aspect of the present invention, the present invention provides a vaccine composition for preventing African swine fever including a combination of African swine fever virus antigen proteins as an active ingredient, wherein the combination of ASFV antigen proteins is a combination of at least one selected from the group consisting of Lectin, CD2v, p72, p54, p30, p15, p35, E199L and/or F317L protein. Preferably, the combination of ASFV antigen proteins may be a combination of at least one selected from the group consisting of Lectin, CD2v, p72, p54 and p30. Most preferably, the combination of ASFV antigen proteins consists of Lectin, CD2v, p72, p54 and p30. In addition, the present invention provides a pharmaceutical composition for preventing or treating African swine fever, including a combination of African swine fever virus antigen proteins as an active ingredient, wherein the combination of ASFV antigen proteins is a combination of at least one selected from the group consisting of Lectin, CD2v, p72, p54, p30, p15, p35, E199L and/or F317L proteins. Preferably, the pharmaceutical composition is a pharmaceutical composition for preventing African swine fever.

Hereinafter, the description of the vaccine composition of the present invention is equally applied to the pharmaceutical composition of the present invention, and conversely, the description of the pharmaceutical composition of the present invention is equally applied to the vaccine composition of the present invention.

Preferably, when the combination of ASFV antigen proteins includes Lectin protein and CD2v protein, the combination of ASFV antigen proteins may consist of three or more antigen proteins. That is, the composition including the Lectin protein and the CD2v protein may further include other ASFV antigen proteins (*e.g*., p72, P54, and/or p30) in addition to the Lectin protein and the CD2v protein.

Preferably, the ASFV antigen protein included in the vaccine composition according to the present invention is produced by using the recombinant vector according to the present invention. Preferably, the ASFV antigen protein is characterized in that it is produced in a plant by using the recombinant vector according to the present invention.

In addition, the vaccine composition according to the present invention may be characterized in that it does not further include ASFV antigen proteins other than Lectin, CD2v, p72, p54 or p30 recombinant proteins. Preferably, the ASFV antigen protein other than the Lectin, CD2v, p72, p54 or p30 recombinant protein may be at least one selected from the group consisting of p15, p35, E199L and F317L. That is, in the most preferred aspect, the vaccine composition according to the present invention is a vaccine composition including 5 types of recombinant antigen proteins Lectin, CD2v, p54, p72 and p30, and, it is characterized in that the antibody production induction effect for the ASFV antigen, the ASFV infection inhibitory effect and the African swine fever prevention effect are higher than vaccines further including additional antigens (p15, p35, E199L, F317L, *etc.*)*.* For example, the vaccine composition may more rapidly induce antibody production against the ASFV antigens compared to vaccines further including p15, p35, E199L, F317L and the like.

In addition, the present invention provides a method for preventing or treating African swine fever, including the step of administering the vaccine composition or pharmaceutical composition according to the present invention to a subject in need thereof. Preferably, the method for preventing or treating African swine fever according to the present invention is characterized in that ASFV antigen proteins (*e.g.,* p15, p35, E199L and/or F317L) other than lectin, CD2v, p72, p54 or p30 recombinant proteins are not administered to the above subjects.

In an embodiment of the present invention, the vaccine composition may further include an adjuvant. Preferably, the adjuvant may be mineral oil or Emulsigen-based, more preferably, it may be a Drakeol 5 oil-based adjuvant, and most preferably, it may be SEA1, but the present invention is not limited thereto.

As used herein, the term "adjuvant" refers to a substance or composition that is added to vaccines or pharmaceutically active ingredients to increase or affect an immune response. Representatively, it means a carrier or auxiliary substance for an immunogen and/or other pharmacologically active substance or composition. Typically, the term "adjuvant" should be interpreted in a broad sense and refers to a broad range of substances or strategies that can enhance the immunogenicity of an antigen incorporated into or administered with the adjuvant. In addition, adjuvants are not limited thereto, and may be divided into immune potentiators, antigen delivery systems or combinations thereof. Examples of suitable adjuvants may include aluminum hydroxide, Freud's complete or incomplete adjuvant, DEAE dextran, levamisole, PCG and poly I:C or poly A:U. In an exemplary embodiment of the present invention, Drakeol 5 oil-based SEA1 adjuvant was used.

As used herein, the term "vaccine" is a biological agent containing an antigen causing an immune response to a living body, and refers to an immunogen which generates immunity in the living body by injection or orally administrating into a human or animal to prevent an infectious disease. The animal is a human or a non-human animal, and the non-human animal refers to a pig, a cow, a horse, a dog, a goat or sheep, but the present invention is not limited thereto.

As used herein, the term "solubility" refers to a degree of solubilizing a target protein or peptide in a solvent suitable for being administered to the human body. Specifically, the solubility indicates a degree of saturation of a solute in a given solvent at a specific temperature. The solubility may be measured by determining a saturation concentration of a solvent, and for example, the solubility may be determined by adding an excessive amount of solute in a solvent, stirring and filtering the solution, and measuring a concentration using an UV spectrophotometer or HPLC, but the present invention is not limited thereto. High solubility is preferable for isolation and purification of a recombinant protein, and has advantages of inhibition of the aggregation of the recombinant protein and maintenance of physiological or pharmacological activity of the recombinant protein.

The content of the antigen protein in the vaccine composition or pharmaceutical composition of the present invention may be appropriately adjusted according to the symptoms of the disease, the degree of progression of the symptoms, the condition of the patient and the like, and for example, it may be0.0001 to 99.9% by weight or 0.001 to 50% by weight based on the total weight of the composition, but the present invention is not limited thereto. The content ratio is a value based on the dry amount after removing the solvent.

The vaccine composition or pharmaceutical composition according to the present invention may further include a suitable carrier, an excipient and a diluent that are commonly used in the manufacture of pharmaceutical compositions. The excipient may be, for example, one or more selected from the group consisting of a diluent, a binder, a disintegrant, a lubricant, an adsorbent, a moisturizer, a film-coating material and a controlled release additive.

The vaccine composition or pharmaceutical composition according to the present invention may be formulated and used in the form of an external agent such as powder, granule, sustained-release granule, enteric granule, liquid, eye drop, elsilic agent, emulsion, suspension, spirit, troche, perfume, Rimonadese, tablet, sustained-release tablet, enteric tablet, sublingual tablet, hard capsule, soft capsule, sustained-release capsule, enteric capsule, pill, tincture, soft extract, dried extract, liquid extract, injection, capsule, perfusate, plaster, lotion, paste solution, spray, inhalant, patch, sterile injectable solution or aerosol, and the external agent may have a dosage form such as cream, gel, patch, spray, ointment, plaster, lotion agent, liniment agent, paste agent or cataplasma agent.

Examples of carriers, excipients and diluents that may be included in the vaccine composition or pharmaceutical composition according to the present invention include lactose, dextrose, sucrose, oligosaccharide, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methylhydroxybenzoate, propylhydroxybenzoate, talc, magnesium stearate and mineral oil.

When formulated, it is prepared by using diluents or excipients such as commonly used fillers, extenders, binders, wetting agents, disintegrants and surfactants.

As additives of tablets, powders, granules, capsules, pills and troches according to the present invention, excipients such as corn starch, potato starch, wheat starch, lactose, white sugar, glucose, fructose, D-mannitol, precipitated calcium carbonate, synthetic aluminum silicate, dibasic calcium phosphate, calcium sulfate, sodium chloride, sodium hydrogen carbonate, purified lanolin, microcrystalline cellulose, dextrin, sodium alginate, methyl cellulose, sodium carboxymethylcellulose, kaolin, urea, colloidal silica gel, hydroxypropyl starch, hydroxypropyl methylcellulose (HPMC) 1928, HPMC 2208, HPMC 2906, HPMC 2910, propylene glycol, casein, calcium lactate and Primojel; and binders such as gelatin, Arabic gum, ethanol, agar powder, cellulose acetate phthalate, carboxymethylcellulose, calcium carboxymethylcellulose, glucose, purified water, sodium caseinate, glycerin, stearic acid, sodium carboxymethylcellulose, sodium methylcellulose, methylcellulose, microcrystalline cellulose, dextrin, hydroxycellulose, hydroxypropyl starch, hydroxymethylcellulose, purified shellac, starch, hydroxypropyl cellulose, hydroxypropyl methylcellulose, polyvinyl alcohol and polyvinylpyrrolidone may be used, and disintegrants such as hydroxypropyl methylcellulose, corn starch, agar powder, methylcellulose, bentonite, hydroxypropyl starch, sodium carboxymethylcellulose, sodium alginate, calcium carboxymethylcellulose, calcium citrate, sodium lauryl sulfate, silicic anhydride, 1-hydroxypropylcellulose, dextran, ion-exchange resin, polyvinyl acetate, formaldehyde-treated casein and gelatin, alginic acid, amylose, guar gum, sodium bicarbonate, polyvinylpyrrolidone, calcium phosphate, gelled starch, Arabic gum, amylopectin, pectin, sodium polyphosphate, ethyl cellulose, white sugar, magnesium aluminum silicate, a di-sorbitol solution and light anhydrous silicic acid; and lubricants such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubri wax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine and light anhydrous silicic acid may be used.

As additives of liquids according to the present invention, water, dilute hydrochloric acid, dilute sulfuric acid, sodium citrate, monostearic acid sucrose, polyoxyethylene sorbitol fatty acid esters (twin esters), polyoxyethylene monoalkyl ethers, lanolin ethers, lanolin esters, acetic acid, hydrochloric acid, ammonia water, ammonium carbonate, potassium hydroxide, sodium hydroxide, prolamine, polyvinylpyrrolidone, ethylcellulose and sodium carboxymethylcellulose may be used.

In syrups according to the present invention, a white sugar solution, other sugars or sweeteners and the like may be used, and as necessary, a fragrance, a colorant, a preservative, a stabilizer, a suspending agent, an emulsifier, a viscous agent or the like may be used.

In emulsions according to the present invention, purified water may be used, and as necessary, an emulsifier, a preservative, a stabilizer, a fragrance or the like may be used.

In suspensions according to the present invention, suspending agents such as acacia, tragacanth, methylcellulose, carboxymethylcellulose, sodium carboxymethylcellulose, microcrystalline cellulose, sodium alginate, hydroxypropyl methylcellulose (HPMC) 1828, HPMC 2906, HPMC 2910 and the like may be used, and as necessary, a surfactant, a preservative, a stabilizer, a colorant and a fragrance may be used.

Injections according to the present invention may include: solvents such as distilled water for injection, a 0.9% sodium chloride solution, Ringer's solution, a dextrose solution, a dextrose+sodium chloride solution, PEG, lactated Ringer's solution, ethanol, propylene glycol, non-volatile oil-sesame oil, cottonseed oil, peanut oil, soybean oil, corn oil, ethyl oleate, isopropyl myristate and benzene benzoate; co-solvents such as sodium benzoate, sodium salicylate, sodium acetate, urea, urethane, monoethylacetamide, butazolidine, propylene glycol, the Tween series, amide nicotinate, hexamine and dimethylacetamide; buffers such as weak acids and salts thereof (acetic acid and sodium acetate), weak bases and salts thereof (ammonia and ammonium acetate), organic compounds, proteins, albumin, peptone and gums; isotonic agents such as sodium chloride; stabilizers such as sodium bisulfite (NaHSO₃) carbon dioxide gas, sodium metabisulfite (Na₂S₂O₅), sodium sulfite (Na₂SO₃), nitrogen gas (N₂) and ethylenediamine tetraacetic acid; sulfating agents such as 0.1% sodium bisulfide, sodium formaldehyde sulfoxylate, thiourea, disodium ethylenediaminetetraacetate and acetone sodium bisulfite; a pain relief agent such as benzyl alcohol, chlorobutanol, procaine hydrochloride, glucose and calcium gluconate; and suspending agents such as sodium CMC, sodium alginate, Tween 80 and aluminum monostearate.

In suppositories according to the present invention, bases such as cacao butter, lanolin, Witepsol, polyethylene glycol, glycerogelatin, methylcellulose, carboxymethylcellulose, a mixture of stearic acid and oleic acid, Subanal, cottonseed oil, peanut oil, palm oil, cacao butter+cholesterol, lecithin, lanette wax, glycerol monostearate, Tween or span, imhausen, monolan(propylene glycol monostearate), glycerin, Adeps solidus, buytyrum Tego-G, Cebes Pharma 16, hexalide base 95, cotomar, Hydrokote SP, S-70-XXA, S-70-XX75(S-70-XX95), Hydrokote 25, Hydrokote 711, idropostal, massa estrarium (A, AS, B, C, D, E, I, T), masa-MF, masupol, masupol-15, neosuppostal-N, paramount-B, supposiro (OSI, OSIX, A, B, C, D, H, L), suppository base IV types (AB, B, A, BC, BBG, E, BGF, C, D, 299), suppostal (N, Es), Wecoby (W, R, S, M, Fs) and tegester triglyceride matter (TG-95, MA, 57) may be used.

Solid preparations for oral administration include tablets, pills, powders, granules, capsules and the like, and such solid preparations are formulated by mixing the composition with at least one excipient, for example, starch, calcium carbonate, sucrose, lactose, gelatin and the like. In addition to simple excipients, lubricants such as magnesium stearate and talc are also used.

Examples of liquid preparations for oral administration include suspensions, liquids for internal use, emulsions, syrups and the like, and these liquid preparations may include, in addition to simple commonly used diluents, such as water and liquid paraffin, various types of excipients, for example, a wetting agent, a sweetener, a fragrance, a preservative and the like. Preparations for parenteral administration include an aqueous sterile solution, a non-aqueous solvent, a suspension, an emulsion, a freeze-dried preparation and a suppository. Non-limiting examples of the non-aqueous solvent and the suspension include propylene glycol, polyethylene glycol, a vegetable oil such as olive oil and an injectable ester such as ethyl oleate.

The vaccine composition or the pharmaceutical composition according to the present invention is administered in a pharmaceutically effective amount. In the present invention, "the pharmaceutically effective amount" refers to an amount sufficient to treat diseases at a reasonable benefit/risk ratio applicable to medical treatment, and an effective dosage level may be determined according to factors including types of diseases of patients, the severity of disease, the activity of drugs, sensitivity to drugs, administration time, administration route, excretion rate, treatment period and simultaneously used drugs, and factors well known in other medical fields.

The vaccine composition or pharmaceutical composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this may be easily determined by those of ordinary skill in the art.

The vaccine composition or pharmaceutical composition of the present invention may be administered to a subject via various routes. All administration methods can be predicted, and the pharmaceutical composition may be administered via, for example, oral administration, subcutaneous injection, intravenous injection, intramuscular injection, intrathecal (space around the spinal cord) injection, sublingual administration, administration via the buccal mucosa, intrarectal insertion, intravaginal insertion, ocular administration, intra-aural administration, intranasal administration, inhalation, spraying via the mouth or nose, transdermal administration, percutaneous administration or the like. Preferably, the composition according to the present invention may be intramuscularly administered.

The vaccine composition or pharmaceutical composition of the present invention is determined depending on the type of a drug, which is an active ingredient, along with various related factors such as a disease to be treated, administration route, the age, gender and body weight of a patient, and the severity of diseases. Specifically, the effective amount of the composition according to the present invention may vary depending on the patient's age, sex and body weight, and generally, 0.001 to 150 mg of the composition and preferably, 0.01 to 100 mg of the composition, per 1 kg of the body weight, may be administered daily or every other day or may be administered once to three times a day. However, since the effective amount may be increased or decreased depending on the administration route, the severity of obesity, gender, body weight, age and the like, the dosage is not intended to limit the scope of the present invention in any way.

As used herein, the term "subject" refers to a subject in need of treatment of a disease, and more specifically, refers to a mammal such as a human or a non-human primate, a mouse, a rat, a dog, a cat, a horse and a cow. For example, as used herein, the term "individual" refers to a subject to whom the recombinant African swine fever antigen protein of the present invention can be administered, and there are no restrictions on the subject.

As used herein, the term "administration" means providing a given composition of the present invention to a subject by any suitable method.

As used herein, the term "prevention" refers to any action that suppresses or delays the onset of a desired disease, and the term "treatment" refers to any action in which the target disease and its metabolic abnormalities are improved or beneficially changed by administration of the pharmaceutical composition according to the present invention, and the term "amelioration" refers to any action that reduces a parameter related to a target disease, for example, the severity of a symptom, by administration of the composition according to the present invention. For example, as used herein, the term "prevention" refers to any action to suppress or delay the onset of African swine fever by administration of the recombinant African swine fever antigen protein according to the present invention. Further, in the present specification, the term "treatment" refers to any action in which the symptoms of African swine fever are alleviated or beneficially changed by administration of the recombinant African swine fever antigen protein according to the present invention.

In order to further describe, the vaccine composition of the present invention may be used by being formulated in the form of an oral formulation such as powder, granules, a tablet, a capsule, a suspension, an emulsion, a syrup and an aerosol, and a sterile injection solution, according to a typical method. When the composition is prepared, the composition may be prepared by using a commonly used diluent or excipient, such as a filler, an extender, a binder, a wetting agent, a disintegrant and a surfactant. A solid formulation for oral formulation includes a tablet, a pill, powder, granules and the like, and the solid formulation may be prepared by mixing at least one excipient, for example, starch, calcium carbonate, sucrose or lactose, gelatin and the like with a lecithin-like emulsifier. Further, in addition to simple excipients, lubricants such as magnesium stearate and talc may also be used. As a liquid formulation for oral administration, a suspension, a liquid for internal use, an emulsion, a syrup and the like may be used, and in addition to water and liquid paraffin which are simple commonly used diluents, various excipients, for example, a wetting agent, a sweetener, an aromatic, a preservative and the like may be included. Examples of a formulation for parenteral administration include an aqueous sterile solution, a non-aqueous solvent, a suspension, an emulsion and a freeze-dried preparation. As the non-aqueous solvent and the suspension, it is possible to use propylene glycol, polyethylene glycol, a vegetable oil such as olive oil, an injectable ester such as ethyl oleate and the like.

The route of administration of the vaccine composition according to the present invention includes, but is not limited to, oral, intravenous, intramuscular, intraarterial, intramedullary, intrathecal, intracardiac, transdermal, subcutaneous, intraperitoneal, intranasal, intestinal, topical, sublingual or rectal routes. Oral or parenteral administration is preferred. As used herein, the term "parenteral" includes subcutaneous, intradermal, intravenous, intramuscular, intraarticular, intrasynovial, intrastemal, intrathecal, intralesional and intracranial injection or infusion techniques. The vaccine composition of the present invention may also be administered in the form of a suppository for rectal administration.

The dosage of the vaccine composition or pharmaceutical composition according to the present invention is selected in consideration of the age, body weight, sex, physical condition and the like of an individual. The amount required to induce an immunoprotective response in an individual without particular side effects may vary depending on the recombinant protein used as an immunogen and the presence of a random excipient.

Preferably, per 2 mL of the vaccine composition according to the present invention, Lectin, CD2v, p54, p72, p15, p35, E199L and/or F317L p may be respectively included at 10 µg to 1 mg, 10 to 900 µg, 10 µg to 800 µg of. 10 µg to 700 µg, 10 µg to 600 µg, 10 µg to 500 µg, 10 µg to 400 µg, 10 µg to 300 µg, 10 µg to 200 µg, 50 µg to 150 µg, 70 µg to 130 µg, 80 µg to 120 µg, or 90 µg to 110 µg, and p30 may be included at 1 µg to 100 µg, 1 µg to 90 µg, 1 µg to 80 µg, 1 µg to 70 µg, 1 µg to 60 µg, 1 µg to 50 µg, 10 µg to 50 µg, 15 µg to 45 µg, 20 µg to 40 µg, or 25 µg to 35 µg, but the present invention is not limited thereto.

In another aspect of the present invention, the present invention provides a vaccine kit for preventing African swine fever, including the vaccine composition according to the present invention. In addition to the vaccine composition of the present invention, the kit may include, without limitation, tools and reagents that are commonly used in the art for vaccine administration. In addition, the kit may include instructions for describing characteristics or information about the vaccine composition, recombinant vector and the like according to the present invention, and may include instructions for describing the method of preparing the vaccine composition according to the present invention.

In another aspect of the present invention, the present invention provides a method for producing a recombinant ASFV antigen protein, including the following steps:
(S1) transforming a plant with the recombinant vector according to the present invention; and
(S2) isolating and purifying the recombinant antigen protein from the plant or culture medium.

In another aspect of the present invention, the present invention provides a preparation method for a vaccine composition or vaccine kit for preventing African swine fever, including the following steps:
(S1) transforming a plant with the recombinant vector according to the present invention;
(S2) isolating and purifying the recombinant antigen protein from the plant or culture medium; and
(S3) preparing a vaccine composition or vaccine kit by using the isolated and purified recombinant antigen protein.

The detailed description of the 'transformation' of step (S1) is the same as described above. The transformation method of step (S1) is not limited to a specific type, but may be preferably performed through an *Agrobacterium-*mediated transformation method. Specifically, step (S1) may include the steps of transforming an *Agrobacteria* strain with the recombinant vector according to the present invention, culturing the transformed *Agrobacteria* strain; and administering (injecting or inoculating) the cultured strain to a plant. Preferably, the plant may be tobacco.

The step of isolating and purifying the recombinant antigen protein in step (S2) may be performed by applying a protein isolation and purification method that is known in the art without limitation. Preferably, the isolation of the protein may be performed by adding a protein extraction solution to the transformed plant. In addition, purification of the protein may be performed by performing chromatography. Preferably, the chromatography may be affinity chromatography, and the ligand used in this case may be protein A, Ni-IDA or the like, but the present invention is not limited thereto.

In another aspect of the present invention, the present invention provides a feed composition for preventing African swine fever, including a combination of African swine fever virus antigen proteins as an active ingredient, wherein the combination of ASFV antigen proteins includes a combination of at least one selected from the group consisting of Lectin, CD2v, p72, p54 and p30 protein. Preferably, when the combination of ASFV antigen proteins includes Lectin protein and CD2v protein, the combination of ASFV antigen proteins may be characterized in that it consists of three or more antigen proteins.

Preferably, the ASFV antigen protein included in the feed composition according to the present invention is characterized in that it is produced by using the recombinant vector according to the present invention. Preferably, the ASFV antigen protein is characterized in that it is produced in a plant by using the recombinant vector according to the present invention.

In addition, preferably, the feed composition according to the present invention is characterized by including all of Lectin, CD2v, p54, p72 and p30 recombinant antigen proteins as active ingredients, and most preferably, the feed composition is characterized in that it does not further include ASFV antigen proteins (p15, p35, E199L and F317L) other than Lectin, CD2v, p72, p54 or p30 recombinant protein.

Specific examples of the "feed" in the feed composition include by-products such as pork, beef, chicken, as well as corn, rice, general rice straw, wild grass, grass, silage, hay, mountain wild grass and the like, but the present invention not limited thereto, and there is no restriction as long as it is a feed used for raising livestock. Examples of a method for adding Lectin, CD2v, p54, p72 and p30 recombinant antigen proteins according to the present invention to the feed and blending the resulting mixture include a method such as mechanical mixing, adsorption and occlusion, but the present invention is not limited thereto.

Hereinafter, preferred examples are presented to aid understanding of the present invention. However, the following examples are provided to more easily understand the present invention, and the content of the present invention is not limited by the following examples.

### [Example]

### Example 1. Preparation of recombinant vector expressing antigens of African swine fever virus

As shown in the cleavage map of FIG. 1, recombinant plant expression vectors were constructed to express the African swine fever virus antigen proteins Lectin, CD2v, p72, p54, p30, p15, p35, E199L or F317L in plants. More specifically, genetic information on the Lectin, CD2v, p72, p54, p30, p15, p35, E199L and F317L proteins of the African swine fever virus was obtained, and the Lectin protein coding gene (SEQ ID NO: 2), the CD2v protein coding gene (SEQ ID NO: 4), the p72 protein coding gene (SEQ ID NO: 6), the p54 protein coding gene (SEQ ID NO: 8), the p30 protein coding gene (SEQ ID NO: 10), the p15 protein coding gene (SEQ ID NO: 12), the p35 protein coding gene (SEQ ID NO: 14), the E199L protein coding gene (SEQ ID NO: 16) and the F317L protein coding gene (SEQ ID NO: 18) were respectively synthesized with sequences that are optimized for expression in plants.

The specific composition of each recombinant vector is as follows:

### Recombinant vector for expressing Lectin antigen protein

The CaMV 35S promoter gene and the HSP terminator were inserted into the pCAMBIA1300 vector, between which a polynucleotide encoding a new chaperone binding protein (NB) signal peptide (SEQ ID NO: 20), a polynucleotide encoding a polyhistidine tag (SEQ ID NO: 26), a polynucleotide encoding the Lectin antigen recombinant protein of African swine fever virus (SEQ ID NO: 2), a polynucleotide encoding the pFc2 (porcine Fc) fragment (SEQ ID NO: 28) and a polynucleotide encoding an HDEL (His-Asp-Glu-Leu) peptide (SEQ ID NO: 30) were sequentially linked to construct a plant expression vector for the Lectin antigen protein of African swine fever virus.

### Recombinant vector for expressing CD2v antigen protein

The CaMV 35S promoter gene and the HSP terminator were inserted into the pCAMBIA1300 vector, between which a polynucleotide encoding the NB signal peptide (SEQ ID NO: 20), a polynucleotide encoding the M domain (SEQ ID NO: 24), and a polynucleotide encoding the CD2v antigen recombinant protein of African swine fever virus (SEQ ID NO: 4), a polynucleotide encoding the pFc2 fragment (SEQ ID NO: 28) and a polynucleotide encoding the HDEL peptide (SEQ ID NO: 30) were sequentially linked to construct a plant expression vector for the CD2v antigen protein of African swine fever virus.

### Recombinant vector for expressing p72 antigen protein

The hygromycin resistance gene, which is a selectable marker of the pCAMBIA1300 vector, was substituted with the human calreticulin 1 gene, and the MacT promoter gene in which the 3' terminal nucleotide of the Mac promoter was substituted with T and the *Arabidopsis* RD29B terminator were inserted, between which a polynucleotide encoding a BiP (chaperone binding protein) signal peptide (SEQ ID NO: 22), a polynucleotide encoding the p72 antigen recombinant protein of African swine fever virus (SEQ ID NO: 6), a polynucleotide encoding the pFc2 fragment (SEQ ID NO: 28) and a polynucleotide for encoding the HDEL peptide (SEQ ID NO: 30) were sequentially linked to construct a plant expression vector for the p72 antigen protein of African swine fever virus.

### Recombinant vector for expressing p54 antigen protein

The CaMV 35S promoter gene and the HSP terminator were inserted into the pCAMBIA1300 vector, between which a polynucleotide encoding the NB signal peptide (SEQ ID NO: 20), a polynucleotide encoding the p54 antigen recombinant protein of African swine fever virus (SEQ ID NO: 8), a polynucleotide encoding the pFc2 fragment (SEQ ID NO: 28) and a polypeptide encoding the HDEL peptide (SEQ ID NO: 30) were sequentially linked to construct a plant expression vector for the p54 antigen protein of African swine fever virus.

### Recombinant vector for expressing p30 antigen protein

The CaMV 35S promoter gene and the HSP terminator were inserted into the pCAMBIA1300 vector, between which a polynucleotide encoding the NB signal peptide (SEQ ID NO: 20), a polynucleotide encoding the p30 antigen recombinant protein of African swine fever virus (SEQ ID NO: 10), a polynucleotide encoding a polyhistidine-tag (SEQ ID NO: 26) and a polynucleotide encoding the HDEL peptide (SEQ ID NO: 30) were sequentially linked to construct a plant expression vector for the p30 antigen protein of African swine fever virus.

### Recombinant vector for expressing p15 antigen protein

The CaMV 35S promoter gene and the HSP terminator were inserted into the pCAMBIA1300 vector, between which a polynucleotide encoding the NB signal peptide (SEQ ID NO: 20), a polynucleotide encoding the p15 antigen recombinant protein of African swine fever virus (SEQ ID NO: 12), a polynucleotide encoding the pFc2 fragment (SEQ ID NO: 28) and a polynucleotide encoding the HDEL peptide (SEQ ID NO: 30) were sequentially linked to construct a plant expression vector for the p15 antigen protein of African swine fever virus.

### Recombinant vector for expressing P35 antigen protein

The CaMV 35S promoter gene and the HSP terminator were inserted into the pCAMBIA1300 vector, between which a polynucleotide encoding the NB signal peptide(SEQ ID NO: 20), a polynucleotide encoding the p35 antigen recombinant protein of African swine fever virus (SEQ ID NO: 14), a polynucleotide encoding the pFc2 fragment (SEQ ID NO: 28) and a polynucleotide encoding the HDEL peptide (SEQ ID NO: 30) were sequentially linked to construct a plant expression vector for the p35 antigen protein of African swine fever virus.

### Recombinant vector for expressing E199L antigen protein

The hygromycin resistance gene, which is a selectable marker of the pCAMBIA1300 vector, was substituted with the turnip crinkle virus-coat protein(TCV-CP) gene, and the MacT promoter gene in which the 3' terminal nucleotide of the Mac promoter was substituted with T and the *Arabidopsis* RD29B terminator were inserted, between which a polynucleotide encoding the NB signal peptide (SEQ ID NO: 20), a polynucleotide encoding the E199L antigen recombinant protein of African swine fever virus (SEQ ID NO: 16), a polynucleotide encoding the pFc2 fragment (SEQ ID NO: 28) and a polynucleotide (SEQ ID NO: 30) encoding the HDEL peptide were sequentially linked to construct a plant expression vector for the E199L antigen protein of African swine fever virus.

### Recombinant vector for expressing F317L antigen protein

The hygromycin resistance gene, which is a selectable marker of the pCAMBIA1300 vector, was substituted with the turnip crinkle virus-coat protein (TCV-CP) gene, and the MacT promoter gene in which the 3' terminal nucleotide of the Mac promoter was substituted with T and the *Arabidopsis* RD29B terminator were inserted, between which a polynucleotide encoding the NB signal peptide (SEQ ID NO: 20), a polynucleotide encoding the F317L antigen recombinant protein of African swine fever virus (SEQ ID NO: 18), a polynucleotide encoding the pFc2 fragment (SEQ ID NO: 28) and a polynucleotide (SEQ ID NO: 30) encoding the HDEL peptide were sequentially linked to construct a plant expression vector for the F317L antigen protein of African swine fever virus.

### Example 2. Confirmation of expression of recombinant ASF virus antigen proteins

### 2-1. Transient expression of plant expression vectors

Each of the plant expression recombinant vectors of the African swine fever antigen proteins (Lectin, CD2v, p72, p54, p30, p15, p35, E199L and F317L) that were prepared in Example 1 was transformed by using electroporation to the *Agrobacterium* LBA4404 strain. After transformed Agrobacteria were cultured in 5 mL of YEP liquid medium (10 g of yeast extract, 10 g of peptone, 5 g of NaCl, 50 mg/L of kanamycin, 25 mg/L of rifampicin) at 28°C with shaking for 16 hours, and 1 mL of primary culture was inoculated into 50 mL of new YEP medium, and it cultured with shaking at 28°C for 6 hours. After the Agrobacteria cultured in this way were collected by centrifugation (7,000 rpm, 4°C, 5 minutes), they were suspended in infiltration buffer [10 mM MES (pH 5.7), 10 mM MgCl₂, 200 µM acetosyringone] such that the absorbance (O.D) value was 1.0 at a wavelength of 600 nm. Agro-infiltration was performed by injecting the Agrobacteria suspension into the back of *Nicotiana benthamiana* leaves by using a syringe with the needle removed.

### 2-2. Isolation and purification of antigen proteins of ASF virus

In order to isolate and purify recombinant proteins from *Nicotiana benthamiana* leaves that were prepared in Example 2-1, a protein extraction solution was added to each recombinant protein-expressing *Nicotiana benthamiana* leaf, and after the tissue was disrupted with a blender, protein extract was recovered by centrifugation at 13,000 rpm at 4°C for 30 minutes. In order to isolate and purify each ASF virus antigen protein from the extract, affinity chromatography was performed by using a column filled with protein A-sepharose resin. The column was filled with Protein A resin and then equilibrated with a washing solution. The recovered protein extract was applied to the column and equilibrated, the resin was washed by flowing a washing solution, and each recombinant protein was eluted with an elution solution. The elution solution including the recombinant antigen protein was neutralized to an appropriate pH by adding a neutralizing solution, and then buffer replacement and concentration were performed by using a filter with a 30 kDa cutoff. The concentration of the isolated and purified recombinant antigen protein was determined through electrophoresis (SDS-PAGE) and Coomassie staining.

In the case of p30 protein, it was isolated and purified through the following process: The protein extract solution was added to the leaves of *Nicotiana bentamia* expressing p30, the tissue was disrupted with a blender, and it was centrifuged at 13,000 rpm at 4°C for 30 minutes to recover protein extract. In order to isolate and purify p30 from the extract, affinity chromatography was performed by using a column filled with Ni-IDA resin. First of all, the column was filled with Ni-IDA resin and then equilibrated with a washing solution. The recovered protein extract was applied to the column, equilibrated and washed with a washing solution to wash the resin, and p30 protein was eluted with an elution solution. The elution solution including the p30 protein was subjected to buffer exchange and concentration by using a 10 kDa cutoff filter. After electrophoresis (SDS-PAGE), the concentration of the isolated and purified p30 protein was confirmed by Coomassie staining.

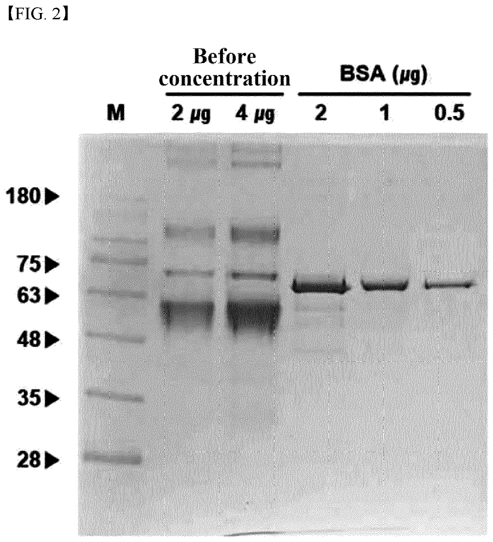

As a result, as shown in FIGS. 2 to 10b, it was confirmed that all of the African swine fever virus antigen proteins according to the present invention were well purified without significant alteration or modification compared to the original proteins. These results verify that no problem was found that could reduce production efficiency due to mutation of the sugar structure when the protein was expressed in plants, confirming that the African swine fever virus recombinant antigen proteins according to the present invention can be produced well in plants.

The isolation and purification conditions and the results for each recombinant antigen protein are as follows:

### Lectin antigen protein (Molecular Weight: 40651.82; FIG. 2)

(1) Protein extraction conditions: 1 kg of *Nicotiana bentamia* leaves, 2 L of protein extract
(2) Protein isolation and purification: Resin down for 20 minutes after binding for 30 minutes in a column filled with about 80 mL of Protein A resin
   - Extraction buffer/washing buffer: 50 mM Tris-Cl (pH 7.2), 100 mM NaCl, 100 mM Sodium sulfite (-W), 0.5% Triton X-100 (-W2), 1.5% PVPP (-W)
   - Elution buffer: 50 mM Sodium citrate (pH 3.0), 100 mM NaCl
   - Neutralization Buffer: 1 M Tris (neutralized to pH 7.5)
   - Final buffer: 50 mM Sodium citrate, about 150 mM Tris-Cl, 100 mM NaCl, pH 7.5
(3) Final protein concentration (based on Nanodrop): 1.02 µg/µL
(4) Final yield (based on Nanodrop): 54 mg/kg

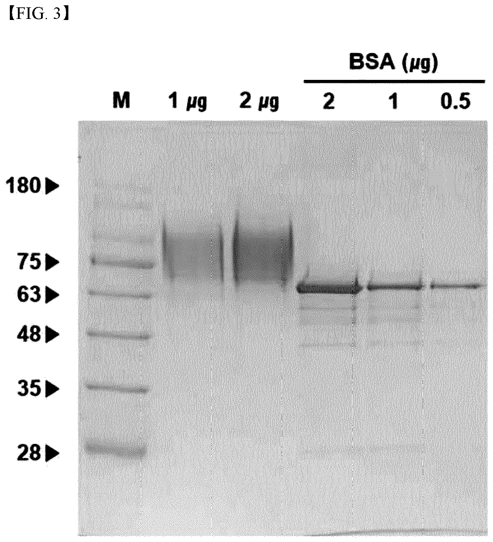

### CD2v antigen protein (Molecular Weight: 55214.70; FIG. 3)

(1) Protein extraction conditions: 1 kg of *Nicotiana bentamia* leaves, 2 L of protein extract
(2) Protein isolation and purification: Resin down for 20 minutes after binding for 60 minutes in a column filled with about 100 mL of Protein A resin
   - Extraction buffer/washing buffer: 100 mM Tris-Cl (pH 7.5), 154 mM NaCl, 0.5% Triton X-100 (-W2), 100 mM Sodium sulfite (-W), 1.5% PVPP (-W)
   - Elution buffer: 50 mM Sodium citrate (pH 3.0), 154 mM NaCl
   - Neutralization Buffer: 1 M Tris (neutralized to pH 7.5)
   - Final buffer: 50 mM Sodium citrate, 154 mM NaCl, add 1M Tris-HCl until pH 7.5.
(3) Final protein concentration (based on Nanodrop): 1.2 µg/µL
(4) Final yield (based on Nanodrop): 68.67 mg/kg

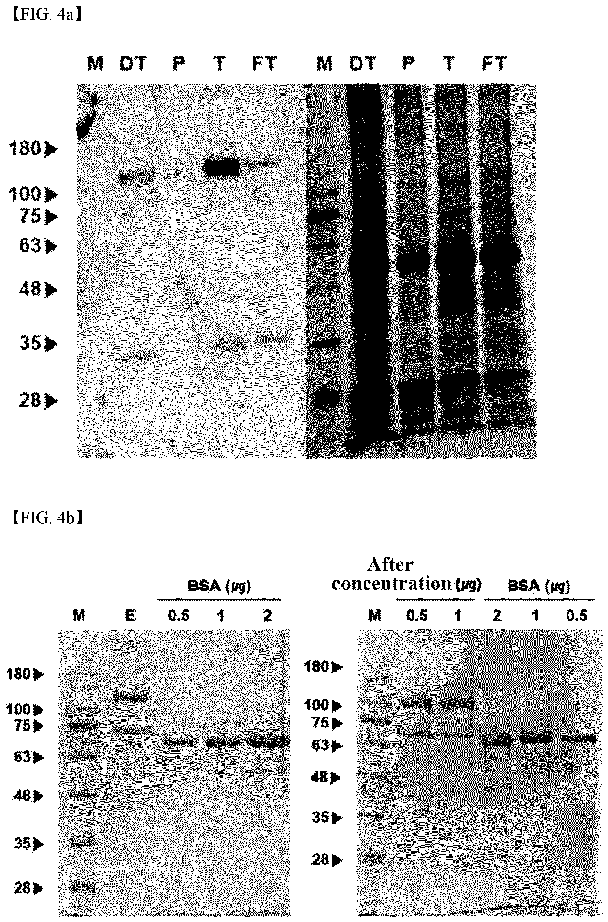

### p72 antigen protein (Molecular Weight: 100405.77 (provided that it was increased by 14 kDa by 7 glycosylation sites); FIGS. 4a and 4b)

(1) Protein extraction conditions: 1 kg of *Nicotiana bentamia* leaves, 2 L of protein extract
(2) Protein isolation and purification: Resin down for 20 minutes after binding for 60 minutes in a column filled with about 100 mL of Protein A resin
   - Extraction buffer/washing buffer: 100 mM Tris-Cl (pH 7.5), 154 mM NaCl, 0.5% Triton X-100 (-W2), 100 mM Sodium sulfite (-W), 1.5% PVPP (-W)
   - Elution buffer: 50 mM Sodium citrate (pH 3.0), 154 mM NaCl
   - Neutralization buffer: 1.5 M Tris (neutralized to pH 7.5)
   - Final buffer: 50 mM Sodium citrate, 154 mM NaCl, add 1M Tris-HCl until pH 7.5.
(3) Final protein concentration (based on Nanodrop): 0.57 µg/µL
(4) Final yield (based on Nanodrop): 7.06 mg/kg

### p54 antigen protein (Molecular Weight: 43956.09 (provided that it was

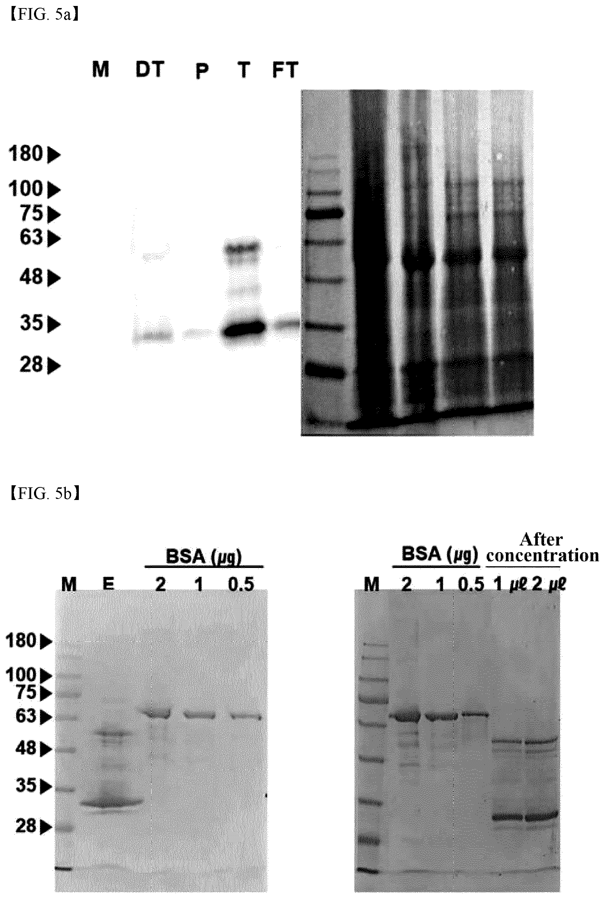

### increased by 2 kDa by one glycosylation site); FIGS. 5a and 5b)

(1) Protein extraction conditions: 0.1 kg of *Nicotiana bentamia* leaves, 0.2 L of protein extract
(2) Protein isolation and purification: Resin down for 20 minutes after binding for 70 minutes in a column filled with about 10 mL of protein A resin
   - Extraction buffer: 100 mM Tris-Cl (pH 7.4), 154 mM NaCl, 0.5% Triton X-100 (-W2), 100 mM Sodium sulfite, 1.5% PVPP, 0.5x PI
   - Washing buffer: 100 mM Tris-Cl (pH 7.4), 154 mM NaCl, 0.5% Triton X-100 (-W2)
   - Elution buffer: 50 mM Sodium citrate (pH 3.0), 154 mM NaCl
   - Final buffer: 1 N NaOH (add 1M Tris-HCl until pH 7.4)
(3) Final protein concentration (based on Nanodrop): 1 mg/mL
(4) Final yield (based on Nanodrop): 71 mg/kg

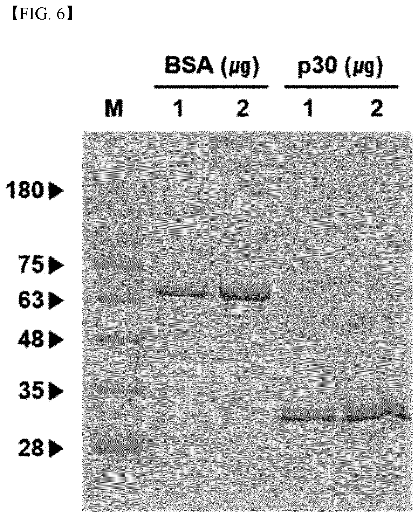

### p30 antigen protein (Molecular Weight: 22993.95; FIG. 6)

(1) Protein extraction conditions: 1 kg of Nicotiana bentamia leaves, 2 L of protein extract
(2) Protein isolation and purification: Resin down for 20 minutes after binding for 60 minutes in a column filled with about 100 mL of Protein A resin
   - Extraction buffer: 50 mM Tris-Cl (pH 8.0), 300 mM NaCl, 10 mM Imidazole, 0.5% Triton X-100, 50 mM Glycine, 100 mM Na 2 SO 3, 10 mM Ascorbic Acid, 1.5% PVPP
   - Washing buffer: 50 mM Tris-Cl (pH 7.4), 300 mM NaCl, 10 mM Imidazole (W1,2), 100 mM Imidazole (W3), 0.5% Triton X-100 (W1)
   - Elution buffer: 50 mM Tris-Cl (pH 7.4), 300 mM NaCl, 250 mM Imidazole
   - Final buffer: 50 mM Tris-Cl pH 7.4, 300 mM NaCl, 50 mM KCI
(3) Final protein concentration (based on Nanodrop): 0.3 µg/µL (volume: 93 mL)
(4) Final yield (based on Nanodrop): 27.9 mg/kg

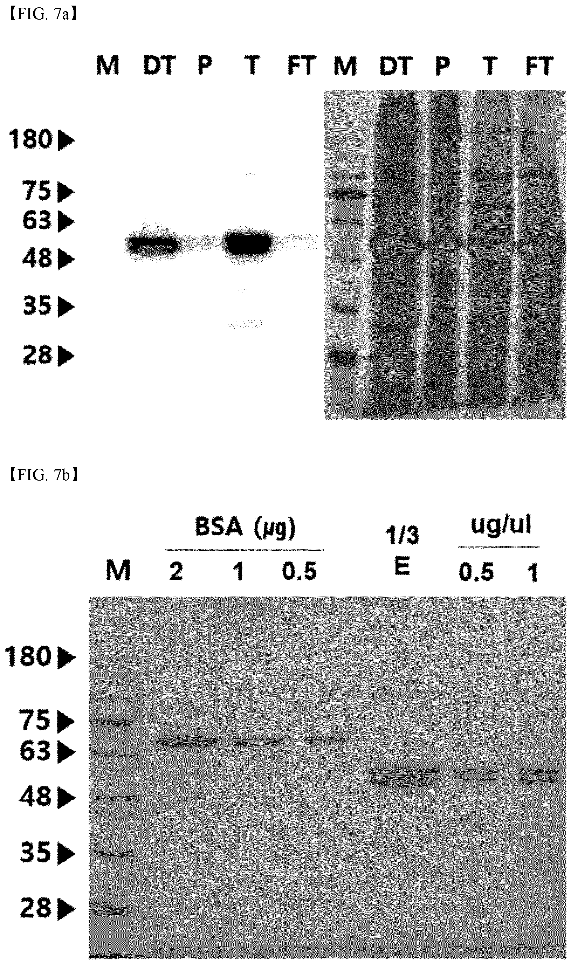

### p15 antigen protein (Molecular Weight: 44114.34; FIGS. 7a and 7b)

(1) Protein extraction conditions: 1.5 kg of Nicotiana bentamia leaves, 1 L of protein extract
(2) Protein isolation and purification: Resin down for 20 minutes after binding for 60 minutes in a column filled with about 100 mL of Protein A resin
   - Extraction buffer/washing buffer: 50 mM Tris-Cl (pH 7.2), 100 mM NaCl, 0.5% Triton X-100 (-W2), 100 mM Sodium sulfite (-W), 1.5% PVPP (-W), 1 mM PMSF
   - Elution buffer: 50 mM Sodium citrate (pH 3.0), 100 mM NaCl
   - Neutralization buffer: 0.5 M NaOH (neutralized to pH 7.5)
   - Final buffer: 50 mM Sodium citrate, 100 mM NaCl, add 1M Tris-HCl until pH 7.5.
(3) Final protein concentration (based on Nanodrop): 0.5 mg/mL
(4) Final yield (based on Nanodrop): 98.6 mg/kg

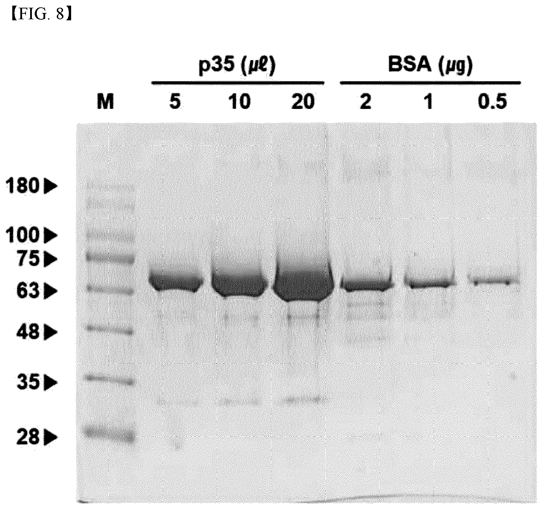

### p35 antigen protein (Molecular Weight: 61323.42; FIG. 8)

(1) Protein extraction conditions: 0.5 kg of *Nicotiana bentamia* leaves, 1 L of protein extract
(2) Protein isolation and purification: Resin down for 20 minutes after binding for 60 minutes in a column filled with about 100 mL of Protein A resin
   - Extraction buffer/washing buffer: 50 mM Tris-Cl (pH 7.2), 100 mM NaCl, 0.5% Triton X-100 (-W2), 100 mM Sodium sulfite (-W), 15g PVPP (-W)
   - Elution buffer: 50 mM Sodium citrate (pH 3.0), 100 mM NaCl
   - Neutralization Buffer: 1 M Tris (neutralized to pH 7.5)
   - Final buffer: 50 mM Sodium citrate, 100 mM NaCl, add 1M Tris-HCl until pH 7.5.
(3) Final protein concentration (based on Nanodrop): 0.53 µg/µL (volume: about 110 ml)
(4) Final yield (based on Nanodrop): 116.6 mg/kg

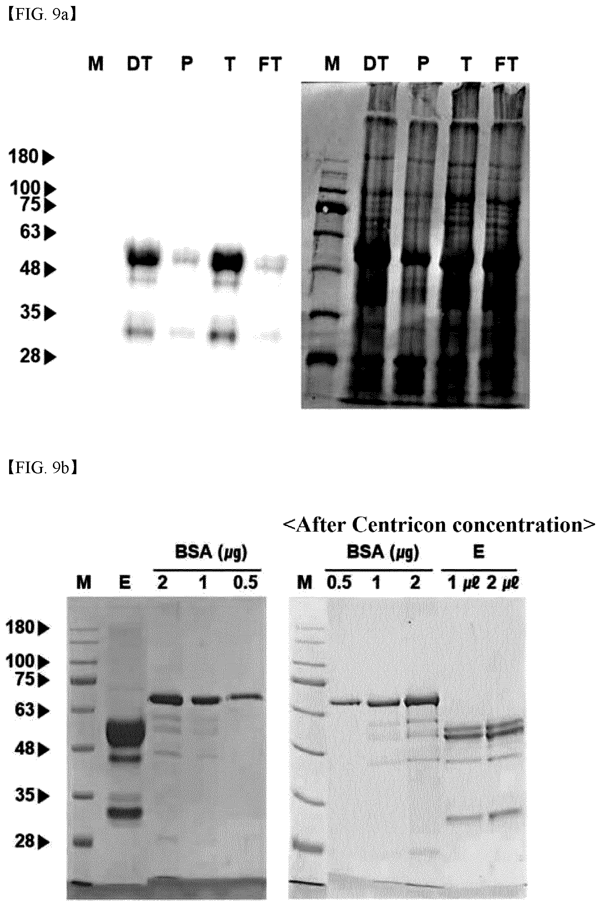

### E199L antigen protein (Molecular Weight: 46485.20 (provided that it was increased by 4 kDa by two glycosylation sites); FIGS. 9a and 9b)

(1) Protein extraction conditions: 1 kg of Nicotiana bentamia leaves, 2 L of protein extract
(2) Protein isolation and purification: Resin down for 20 minutes after binding for 60 minutes in a column filled with about 100 mL of Protein A resin
   - Extraction buffer: 100 mM Tris-Cl (pH 7.2), 154 mM NaCl, 0.5% Triton X-100, 100 mM Sodium sulfite, 1.5% PVPP, 1 mM PMSF (DMSO)
   - Washing buffer: 100 mM Tris-Cl (pH 7.2), 154 mM NaCl, 0.5% Triton X-100 (-W2)
   - Elution buffer: 50 mM Sodium citrate (pH 3.0), 154 mM NaCl
   - Neutralization buffer: 0.2 N NaOH (neutralized to pH 7.5)
(3) Final protein concentration (based on Nanodrop): 1 mg/mL
(4) Final yield (based on Nanodrop): 70.3 mg/kg

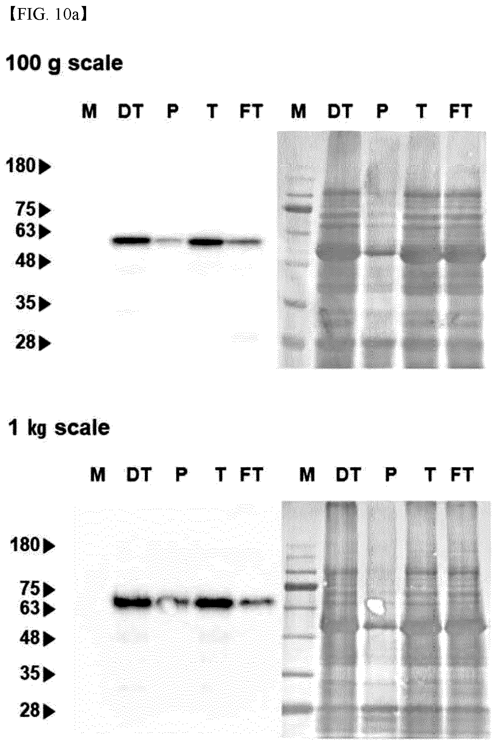

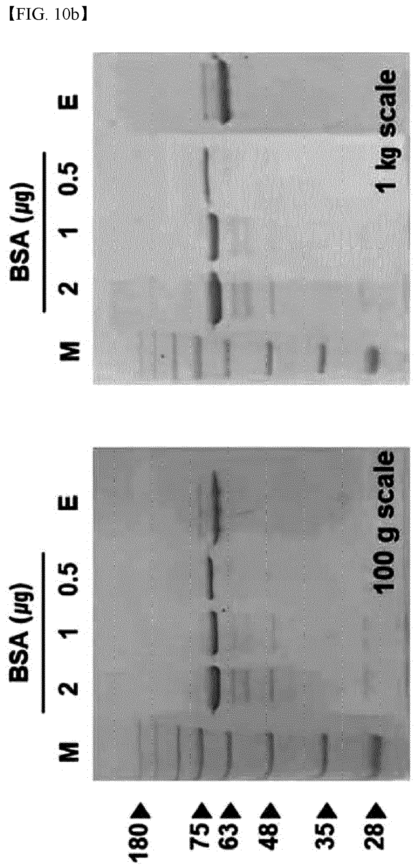

### F317L antigen protein (Molecular Weight: 62777.73 (provided that it was increased by 6 kDa by 3 glycosylation sites); FIGS. 10a and 10b)

(1) Protein extraction conditions: 100 g of *Nicotiana benthamia* leaves and 200 mL of protein extract; or 1 kg of *Nicotiana benthamia* leaves and 2 L of protein extract
(2) Protein isolation and purification: Resin down for 20 minutes after binding in a column filled with about 10 mL of protein A resin for 60 minutes
   - Extraction buffer: 100 mM Tris-Cl (pH 7.5), 154 mM NaCl, 0.5% Triton X-100, 100 mM Sodium sulfite, 1.5% PVPP, 1 mM PMSF (DMSO)
   - Washing buffer: 100 mM Tris-Cl (pH 7.5), 154 mM NaCl, 0.5% Triton X-100 (-W2)
   - Elution buffer: 50 mM Sodium citrate (pH 3.0), 154 mM NaCl
   - Neutralization buffer: 0.2 N NaOH (neutralized to pH 7.5)
(3) Final protein concentration (based on Nanodrop): 1 mg/mL
(4) Final yield (based on Nanodrop): 14 mg/kg

### Example 3. Confirmation of ASF virus defensive effect by 5-antigen vaccine or 9-antigen vaccine

In order to confirm the ASF virus infection preventive effect and the safety of the vaccine by administration of the ASF virus antigen proteins that were isolated and purified in Example 2, a total of 18 pigs were divided into 3 groups of 6 pigs as shown in Table 1 below to perform the defensive ability test. Specifically, the animal model was divided into a 5-antigen vaccine (combination of Lectin, CD2v, p54, p72 and p30) treatment group (G1), a 9-antigen vaccine (combination of Lectin, CD2v, p54, p72, p30, p15, p35, E199L and F317L) treatment group (G2) and a PBS treatment control group (G3). The ASFV antigen proteins other than p30 were included in each vaccine at 100 µg and p30 was included at 30 µg, and 2 mL of the vaccine was administered to one pig per inoculation.

**[Table 1]**

| **Group** | **Challenge inoculation method** | **Vaccine (2 mL)** |
|---|---|---|
| G1 (6 pigs) | IM (2 pigs) | Lectin+CD2v+p54+p72+p30 (100µg each except p30, P30 is 30µg), SEA1 |
| | Sentinel (4 pigs) | |
| G2 (6 pigs) | IM (2 pigs) | Lectin+CD2v+p54+p72+p30+p15+p35+E199L+F317L (100µg each except p30, p30 is 30µg), SEA1 |
| | Sentinel (4 pigs) | |
| G3 (6 pigs) | IM (2 pigs) | PBS |
| | Sentinel (4 pigs) | |

The protective ability test schedule is shown in Table 2. A vaccine was prepared by mixing the recombinant protein antigen cocktail with SEA1 adjuvant, and intramuscular injections were performed twice at 3-week intervals, and when 2 weeks elapsed after the secondary injection, two animals from each group were challenged with African swine fever virus (wild-type ASF virus) by intramuscular injection. The remaining four animals in each group were managed in the same breeding room as the pigs that had been intramuscularly inoculated with the African swine fever virus, and were brought into contact with the virus that was released from the challenged pigs to induce horizontal infection (Sentinel). Symptoms were observed every day after challenge inoculation, and pigs that showed the symptoms of infection (temperature above 41.1°C) were euthanized early before the end of the test and serum was collected. Specifically, 3 to 5 mL of serum was obtained from the jugular vein or anterior vena cava (using an 18 to 20G, 1 1/2 needle), and infected tissues such lung, liver, spleen, kidney, lymph nodes and pancreas were isolated.

**[Table 2]**

| **Schedule** | **Animal treatments** | **Primary inoculati on** | | | **Seconda ry inoculati on** | | **Challeng e inoculati on** | | **Finish** |
|---|---|---|---|---|---|---|---|---|---|
| | day post vaccination | 0 | 7 | 14 | 21 | 28 | 35 | 42 | 49 |
| | day post challenge | | | | | | 0 | 7 | 14 |
| sampling | serum (specific Ab against self antigen; ELISA) | Y | Y | Y | Y | Y | Y | Y | Y |
| | serum (Ab against p30, CD2v; Jishu ELISA) | Y | Y | Y | Y | Y | Y | Y | Y |
| | serum (viremia; real-time PCR) | | | | | | Y | Y | Y |
| | PBMC (IFN-γ; ELISA) | | | | | | Y | | Y |
| | lung, spleen, liver, lymph nodes, kidneys, pancreas (viremia; real-time PCR) | | | | | | | | Y |
| * Daily observation after challenge inoculation, temperature measurement-clinical score, body temperature, survival rate | | | | | | | | | |
| * Primary/secondary inoculation: 2 mL each, IM (intramuscular injection) | | | | | | | | | |
| * challenge: 10²HAD₅₀ASFV-Vietnam,IM (2 pigs per group) | | | | | | | | | |

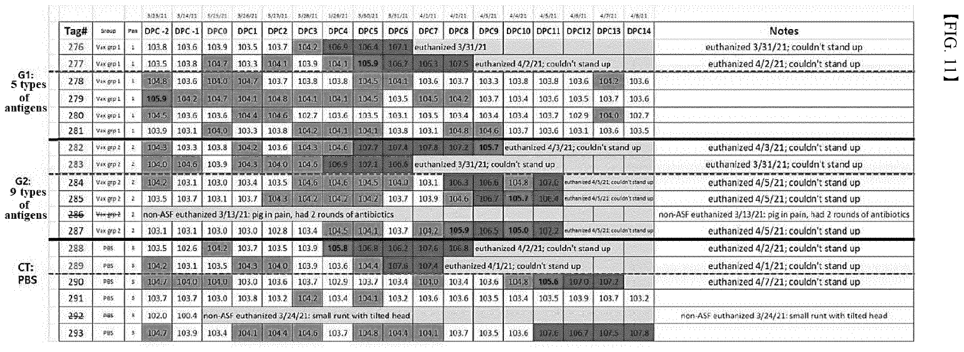

The results of body temperature change and survival rate of pigs according to challenge inoculation are shown in FIG. 11. Two pigs from each group that were directly challenged with African swine fever virus died before and after one week of challenge inoculation. However, all non-challenged pigs in the group (G1) to which the 5-antigen vaccine was administered showed a body temperature of less than 40.5°C, and all survived for 2 weeks. On the other hand, all three non-challenged pigs in the group (G2) to which the 9-antigen vaccine was administered died after 11 days due to high fever, and one of the three non-challenged pigs in the control group (G3) survived without symptoms, but one died on day 13 due to high fever, and the other one had a high fever from day 11, but survived for the 2 weeks set as the experimental period.

These results show that the 5-antigen vaccine (combination of Lectin, CD2v, p54, p72 and p30) according to the present invention has an excellent preventive effect against swine fever virus, and particularly, combining p15, p35, E199L and F317L antigen proteins (i.e., 9-antigen vaccine), which have been used as conventional ASF virus vaccine antigens, rather inhibited the function of the vaccine, suggesting that the African swine fever preventive effect is reduced.

### Example 4. Confirmation of the level of virus (Viremia) in the blood according to challenge inoculation after administration of 5-antigen vaccine or 9-antigen vaccine

Subsequently, the level of virus in the blood (also referred to as "viremia") of pigs that were subjected to the challenge inoculation test of Example 3 was measured to confirm the preventive effect of the vaccine according to the present invention against African swine fever virus.

Specifically, serum was extracted from each pig at time points when 0, 3, 7, 10 and 14 days had elapsed after challenge inoculation to measure the level of viremia, and after performing real-time polymerase chain reaction, the Ct values of each sample were compared. The ASF virus-specific primer sequences used in the experiment are as follows:

**[Table 3]**

| **Primers** | | **SEQ ID NO.** |
|---|---|---|
| *ASFFP1* | 5'-GCGATGATGATTACCTTTGCTTTG-3' | 41 |
| *ASFFP2* | 5'-CGATGATGATTACCTTCGCTTTGA-3' | 42 |
| *ASFVRP1* | 5'-CGATACCACAAGATCAGCCGT-3' | 43 |
| *ASFVRP2* | 5'-CTGATACCACAAGATCAGCCGT-3' | 44 |
| *ASFVRP3* | 5'-GATACCACAAGATCGGCCGT-3' | 45 |
| **Probe** (5'-3') | Texas Red-CACGGGAGGAATACCAACCCAG-BHQ2 | 46 |

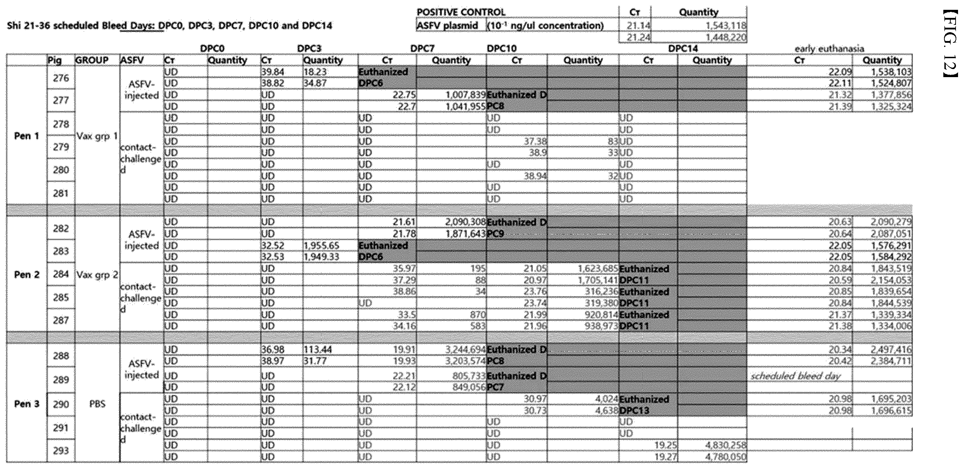

As a result, as shown in FIG. 12, among the pigs (G1) that were administered with the 5-antigen vaccine, pigs that were contact challenged with the ASF virus had very low and undetectable blood levels of the ASF virus until 7 days after inoculation (UD), and on day 10 after inoculation, the virus was detected in some pigs, but at very low levels and was not detected on day 14. On the other hand, among the pigs (G2) that were administered with the 9-antigen vaccine, the ASF virus was detected in pigs that were contact challenged with the ASF virus 7 days after inoculation, and after 10 days had elapsed, the level of viremia increased, and the level of viremia was found to be even higher compared to the control group at the same time point, and thus, it could be confirmed that the virus prevention effect was further reduced compared to not administering the vaccine at all. In addition, as described above, the pigs that were administered the 9-antigen vaccine were euthanized 11 days after the challenge inoculation because their fever symptoms worsened.

The above results demonstrate that the 5-antigen vaccine (combination of Lectin, CD2v, p54, p72 and p30) according to the present invention can achieve an excellent swine fever prevention effect by inhibiting the infection and proliferation of the ASF virus, and particularly show that additionally combining the p15, p35, E199L and F317L antigen proteins (i.e., 9-antigen vaccine), which have been used as conventional ASF virus vaccine antigens, actually inhibits the effectiveness of the vaccine and cannot prevent virus infection and proliferation in the body at all.

### Example 5. Confirmation of anti-ASFV antibody levels after administration of 5-antigen vaccine or 9-antigen vaccine

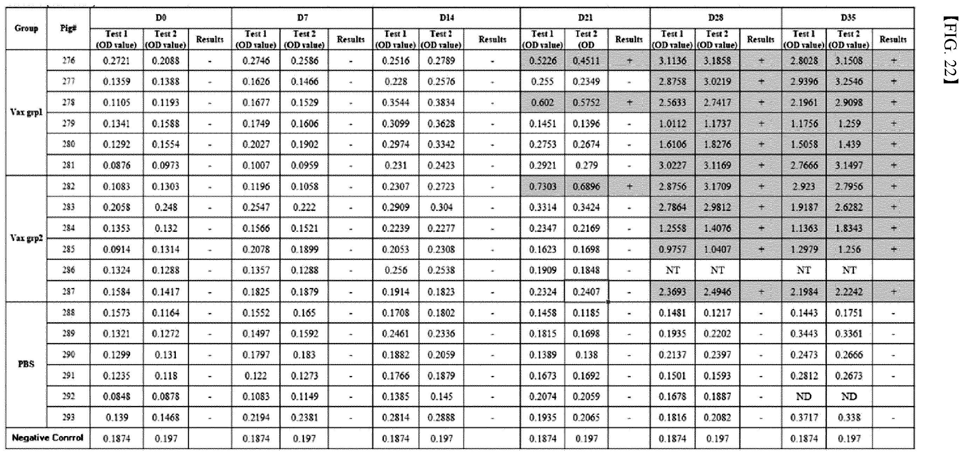

Next, in order to determine the level of anti-ASFV antibodies in serum, the degree of anti-p30 antibody production over time after vaccination was monitored in all subjects by using vaccine antigen-coated plates (performed on days 0, 7, 14, 21, 28 and 35 after vaccination). In the group receiving the 5-antigen vaccine, the levels of anti-p30 antibodies were detected from day 21 after vaccination, and the high levels of anti-p30 antibodies were detected in all subjects that were administered with the vaccine on days 28 and 35 after vaccination, and an average higher antibody response than the group administered with the 9-antigen vaccine was confirmed (FIG. 22).

The above results show that both of the 5-antigen vaccine and 9-antigen vaccine according to the present invention can induce antibody production against the swine fever virus antigens, but particularly show that the 5- type antigen vaccine can induce higher levels of antibody production more quickly than the 9-antigen vaccine.

As described above, the inventors of the present invention constructed vectors expressing African swine fever virus-specific proteins for expression in plants, and when the ASF virus vaccine in which the recombinant proteins were combined was administered to pigs, it was confirmed that the production of antibodies against the ASF virus was stably induced without side effects. In particular, pigs that were administered with the 5-antigen vaccine in which five types of antigens (Lectin, CD2v, p54, p72 and p30) were combined steadily gained body weight even after exposure to the virus, no ASFV viremia or fever was observed, and they showed a 100% survival rate, and thus, it was confirmed that the 5-antigen vaccine can suppress African swine fever by effectively inhibiting the infection and proliferation of the ASF virus in the body. In particular, since it was confirmed that the antibody production inducing effect of the 5-antigen vaccine was excellent compared to the 9-antigen vaccine (Lectin, CD2v, p54, p72, p30, p15, p35, E199L and F317L), this means that the combination of antigen proteins Lectin, CD2v, p54, p72 and p30 can achieve an excellent African swine fever preventive effect compared to conventional vaccines. Therefore, it is expected that the 5-antigen vaccine according to the present invention can be utilized in various ways as a novel African swine fever virus vaccine composition.

The above description of the present invention is for illustrative purposes, and those skilled in the art will understand that it can be easily modified into other specific forms without changing the technical spirit or essential features of the present invention. Therefore, it should be understood that the exemplary embodiments described above are illustrative in all respects and not restrictive.

### [Industrial Applicability]

The recombinant African swine fever virus antigen protein of the present invention is not only effectively expressed in plants, but also has high water solubility, and thus, it is easy to isolate and purify the same, and it also acts as an antigen in the body to show high immunogenicity such that it can be used as a novel African swine fever virus vaccine composition. In particular, the inventors of the present invention confirmed that the vaccine composition including the five types of antigen proteins (Lectin, CD2v, p54, p72 and p30) of the present invention is more effective in preventing African swine fever than the vaccine composition that further includes other antigen proteins (p15, p35, E199L, F317L, *etc.),* and thus, it is expected that the recombinant vector and vaccine composition according to the present invention will be widely utilized in the livestock industry.

## Claims

1. A vaccine composition for preventing African swine fever, comprising:
a combination of African swine fever virus (ASFV) antigen proteins as an active ingredient,
wherein the combination of ASFV antigen proteins is a combination of at least one selected form the group consisting of Lectin, CD2v, p72, p54 and p30 proteins, and
wherein when the combination of ASFC antigen proteins comprises the Lectin protein and the CD2v protein, the combination of ASFV antigen proteins consists of at least three antigen proteins.

2. The vaccine composition of claim 1, wherein the vaccine composition satisfies at least one feature selected from the group consisting of the following:
(a) the Lectin protein comprises the amino acid sequence of SEQ ID NO: 1;
(b) the CD2v protein comprises the amino acid sequence of SEQ ID NO: 3;
(c) the p72 protein comprises the amino acid sequence of SEQ ID NO: 5;
(d) the p54 protein comprises the amino acid sequence of SEQ ID NO: 7; and
(e) the p30 protein comprises the amino acid sequence of SEQ ID NO: 9.

3. The vaccine composition of claim 1, wherein the ASFV antigen protein is produced by using a recombinant vector, and the recombinant vector comprises at least one ASFV protein-encoding polynucleotide selected from the group consisting of the following:
(a) a Lectin-encoding polynucleotide comprising the nucleotide sequence represented by SEQ ID NO: 2;
(b) a CD2v-encoding polynucleotide comprising the nucleotide sequence represented by SEQ ID NO: 4;
(c) a p72-encoding polynucleotide comprising the nucleotide sequence represented by SEQ ID NO: 6;
(d) a p54-encoding polynucleotide comprising the nucleotide sequence represented by SEQ ID NO: 8; and
(e) a p30-encoding polynucleotide comprising the nucleotide sequence represented by SEQ ID NO: 10.

4. The vaccine composition of claim 3, wherein the recombinant vector further comprises at least one selected from the group consisting of the following:
(a) a polynucleotide encoding NB of SEQ ID NO: 19 or BiP of SEQ ID NO: 21;
(b) a polynucleotide encoding the pFc2 fragment of SEQ ID NO: 27;
(c) a polynucleotide encoding the HDEL peptide of SEQ ID NO: 29; and
(d) a polynucleotide encoding the M domain of SEQ ID NO: 23 or the polyhistidine tag of SEQ ID NO: 25.

5. The vaccine composition of claim 3, wherein the ASFV antigen protein is produced in a plant that is transformed with the recombinant vector.

6. The vaccine composition of claim 1, wherein the vaccine composition further comprises an adjuvant.

7. The vaccine composition of claim 6, wherein the adjuvant is mineral oil or Emulsigen-based.

8. A vaccine kit for preventing African swine fever, comprising the vaccine composition according to any one of claims 1 to 7.

9. A method for preventing African swine fever, comprising the step of:
administering the vaccine composition of claim 1 to a non-human animal.

10. A feed composition for preventing African swine fever, comprising:
a combination of African swine fever virus (ASFV) antigen proteins as an active ingredient,
wherein the combination of ASFV antigen proteins is a combination of at least one selected form the group consisting of Lectin, CD2v, p72, p54 and p30 proteins, and
wherein when the combination of ASFC antigen proteins comprises the Lectin protein and the CD2v protein, the combination of ASFV antigen proteins consists of at least three antigen proteins.

11. A recombinant vector for expressing an antigen protein of African swine fever, comprising:
a polynucleotide encoding the p72 protein comprising the amino acid sequence of SEQ ID NO: 5; a polynucleotide encoding the p54 protein comprising the amino acid sequence of SEQ ID NO: 7; a polynucleotide encoding p15 protein comprising the amino acid sequence of SEQ ID NO: 11; a polynucleotide encoding the p35 protein comprising the amino acid sequence of SEQ ID NO: 13; a polynucleotide encoding the E199L protein comprising the amino acid sequence of SEQ ID NO: 15; or a polynucleotide encoding the F317L protein comprising the amino acid sequence of SEQ ID NO: 17,
wherein the recombinant vector is expressed in a plant.

12. The recombinant vector of claim 11, wherein the recombinant vector satisfies at least one feature selected from the group consisting of the following:
(a) the polynucleotide encoding the p72 protein comprises the nucleotide sequence represented by SEQ ID NO: 6;
(b) the polynucleotide encoding the p54 protein comprises the nucleotide sequence represented by SEQ ID NO: 8;
(c) the polynucleotide encoding the p15 protein comprises the nucleotide sequence represented by SEQ ID NO: 12;
(d) the polynucleotide encoding the p35 protein comprises the nucleotide sequence represented by SEQ ID NO: 14;
(e) the polynucleotide encoding the E199L protein comprises the nucleotide sequence represented by SEQ ID NO: 16; and
(f) the polynucleotide encoding the F317L protein comprises the nucleotide sequence represented by SEQ ID NO: 18.

13. The recombinant vector of claim 11, wherein the recombinant vector further comprises a polynucleotide encoding the new chaperone binding protein (NB) of SEQ ID NO: 19 or a polynucleotide encoding the chaperone binding protein (BiP) of SEQ ID NO: 21.

14. The recombinant vector of claim 13, wherein the polynucleotide encoding the NB or BiP is located in the 5' terminal direction of the polynucleotide encoding the p72 protein, p54 protein, p15 protein, p35 protein, E199L protein or F317L protein.

15. The recombinant vector of claim 11, wherein the recombinant vector further comprises a polynucleotide encoding the pFc2 (porcine Fc) fragment of SEQ ID NO: 27.

16. The recombinant vector of claim 15, wherein the polynucleotide encoding the pFc2 fragment is located in the 3' terminal direction of the polynucleotide encoding the p72 protein, p54 protein, p15 protein, p35 protein, E199L protein or F317L protein.

17. The recombinant vector of claim 11, wherein the recombinant vector further comprises a polynucleotide encoding the HDEL (His-Asp-Glu-Leu) peptide of SEQ ID NO: 29.

18. The recombinant vector of claim 17, wherein the polynucleotide encoding the HDEL peptide is located in the 3' terminal direciton of the polynucleotide encoding the p72 protein, p54 protein, p15 protein, p35 protein, E199L protein or F317L protein.

19. The recombinant vector of claim 11, wherein the recombinant vector further comprises a polynucleotide encoding NB of SEQ ID NO: 19 or BiP of SEQ ID NO: 21; a polynucleotide encoding the pFc2 fragment of SEQ ID NO: 27; and a polynucleotide encoding the HDEL peptide of SEQ ID NO: 29.

20. The recombinant vector of claim 19, wherein in the recombinant vector, the polynucleotide encoding NB or BiP;
the polynucleotide encoding the p72 protein, p54 protein, p15 protein, p35 protein, E199L protein or F317L protein;
the polynucleotide encoding the pFc2 fragment; and
the polynucleotide encoding HDEL peptide are sequentially linked.

21. A transformant, which is transformed with the recombinant vector according to any one of claims 11 to 20.

22. The transformant of claim 21, wherein the transformant is a plant.

23. Use of the vaccine composition of claim 1 in the prevention of African swine fever.

24. Use of the vaccine composition of claim 1 in the manufacture of a vaccine for preventing African swine fever.
